# EUROPÄISCHE PATENTSCHRIFT

(11) **EP 1 456 210 B1**
(45) Veröffentlichungstag und Bekanntmachung des Hinweises auf die Patenterteilung: **22.02.2006**
(21) Anmeldenummer: 02796478.2
(22) Anmeldetag: 19.12.2002
(51) Int. Cl.: C07D 487/04, A61K 31/5025, A61K 31/517, C07D 249/00, C07D 241/00

(54) **PYRAZOLYL-SUBSTITUIERTE TRIAZOLOCHINOXALINE**
PYRAZOLYL-SUBSTITUTED TRIAZOLOQUINOXALINES
TRIAZOLOQUINOXALINE SUBSTITUEE PAR PYRAZOLYL

(30) Priorität: 21.12.2001 AT 20252001
(43) Veröffentlichungstag der Anmeldung: 15.09.2004
(73) Patentinhaber: JSW-Research Forschungslabor Gmbh, 8020 Graz (AT)
(72) Erfinder: MATUSZCZAK, Barbara, A-6020 Innsbruck (AT); MÜLLER, Christa, E., 53115 Bonn (DE)
(74) Vertreter: Dungler, Karin
(86) Internationale Anmeldenummer: PCT/AT2002/000361
(87) Internationale Veröffentlichungsnummer: WO 2003/053973

(56) Entgegenhaltungen:
- MATUSZCZAK B, PEKALA E, MÜLLER C.: "1-Substituted 4-[Chloropyrazolyl][1,2,4]triazolo[4,3-a]q uinoxalines:Synthesis and Structure-Activity Reltionships of a New Class of Benzodiazepine and Adenosine Receptor Ligands " ARCH.PHARM.PHARM.MED.CHEM, Bd. 331, 1998, Seiten 163-169, XP001146470 in der Anmeldung erwähnt
- C.E.MÜLLER: "A3 Adenosine Receptor Antagonists" MINI REV.MED.CHEM., Bd. 1, 2001, Seiten 339-348, XP002234892

## Beschreibung

Die Erfindung betrifft pyrazolyl-substituierte Triazolochinoxaline, deren analoge Tetrazolochinoxaline sowie deren pharmazeutisch verträgliche Salze, deren Herstellung sowie deren Verwendung als Arzneimittel zur Behandlung von Erkrankungen im Bereich der Nieren (als K⁺⁻sparende Diuretika, bei akutem Nierenversagen, bei Nephritis, bei hepatorenalem Syndrom), zur Behandlung des Herzens (vorzugsweise bei Herzrhythmusstörungen, Ischämie, Herzinfarkt bzw. Angina pectoris), des ZNS (bei Demenz, Morbus Alzheimer, Angststörungen, Epilepsie, Morbus Parkinson, Schlaganfall, Depressionen, Opiatentzug und komatösen Zuständen), bei Behandlung der Lunge (zur Therapie der Atemwegserkrankungen, beispielsweise Asthma, Bronchitis und Mucoviscidose sowie als Protetektiva bei einer Lungentransplantation). Weiters umfasst die Erfindung Arzneimittel zur Behandlung der Hypertonie, allergischen Hauterkrankungen (Urticaria), Entzündungen, als Immunstimulans, zur Verringerung der Spermienmotilität, woraus sich eine kontrazeptive Wirkung ableitet, sowie als Diagnostika.

### Stand der Technik

Es ist bekannt, dass das Nukleosid Adenosin ein in Säugetieren, einschließlich des Menschen, ubiquitär verbreiteter Modulator ist, der in Form seiner Di- und Triphosphate auf das Engste in den Energiehaushalt eingebunden ist. Adenosin selbst wirkt auf das Nervensystem, das kardiovaskuläre System, das Immunsystem, die Atemwege und den Stoffwechsel ein, wobei diese Wirkungen im allgemeinen von dämpfender Natur sind, d.h. die Wirkungen sind sedierend, gefäßerweiternd, die Herzfrequenz verlangsamend und die Diurese sowie Lipolyse hemmend.

Bisher wurden vier verschiedene Adenosinrezeptoren charakterisiert, welche die Adenylatzyklase über Kopplung an Protein G aktivieren und die Bezeichungen A₁, A_{2A}, A_{2B} und A₃ tragen. Das Zentralnervensystem weist besonders hohe Dichten an Adenosinrezeptoren auf, jedoch finden sich insbesondere A₁- und A₂₈-Rezeptoren in fast allen Gewebetypen. Wegen ihrer vielfältigen Bedeutung in der medizinischen Physiologie sowie der Pharmakologie sind Adenosinrezeptoren vielfach Gegenstand ausführlicher wissenschaftlicher Übersichtsartikel (siehe z.B. K.N. Klotz, *Naunyn Schmiedebergs Arch. Pharmacol.,* **362**, 382-91 (2000); P.G. Baraldi *et al., Med. Res. Rev.,* **20**,103-28 (2000); C.E. Müller, *Farmaco,* **56**, 77-80 (2001) und andere). Während die Rezeptorsubtypen A₁ und A_{2A} hochaffin sind und durch Adenosin bereits in nanomolaren Konzentrationen stimuliert werden, weisen die Subtypen A_{2B} und A₃ geringe (mikromolare) Affinität zum natürlichen Liganden auf. Auf dieser Grundlage wurden Überlegungen einer gestaffelten Aktivierung dieser Rezeptoren durch steigende Adenosinkonzentrationen angestellt, wobei A₁-Rezeptoren die basale Aktivierung bei physiologischen Ruhekonzentrationen bereitstellen und A₃-Rezeptoren in erster Linie bei Ausnahmezuständen mit stark erhöhten Adenosinkonzentrationen, etwa bei ischämischem Schlaganfall oder Herzinfarkt ihre Wirkung entfalten.

Adenosin A₁-Rezeptoren sind also nach dieser Modellvorstellung fast ständig aktiviert und üben eine tonische inhibitorische Kontrolle aus, die durch Antagonisten aufgehoben werden kann; dies ist z.B. die pharmakologische Grundlage der belebenden und entwässemden Wirkung des Coffeins. Adenosin A₁-Antagonisten sind daher als pharmakologische Wirkstoffe in mehrfacher Hinsicht äußerst interessant:
- Im Rahmen der Psychiatrie zur Förderung der Kognition bei Demenzzuständen und als Antidepressiva;
- Im kardiovaskulären und urologischen Bereich als Antihypertonika und Antiarrhythmika;
- Zur Therapie des akuten Nierenversagens, bei welchem Adenosin A₁-Blockade die sekundäre Vasokonstriktion aufheben und die Durchblutung erhöhen kann. Hier kommt dem davon unabhängigen diuretischen Effekt, speziell der Förderung der kaliumsparenden Natriurese, von A₁-Antagonisten zusätzliche Bedeutung zu, da eine Wasser retention zu einer weiter erhöhten Belastung des Kreislaufes führt.
- In der Lunge können Adenosin A₁-Antagonisten einer über Aktivierung von A₁-Rezeptoren vermittelten Bronchokonstriktion durch Entspannung der glatten trachealen Muskulutor entgegenwirken; sie sind daher potentielle Antiasthmatika. Da diese Wirkstoffe dort zudem die Ausschleusung von Chloridionen aus Epithelzellen fördem, wird eine positive Wirkungen auf das Zustandsbild der Mucoviscidose diskutiert.

Bemerkenswerterweise wirkt die Aktivierung von A_{2A}-Rezeptoren im Gehim sowie in der Netzhaut des Auges antagonistisch auf A₁-Rezeptoren. A_{2A}-Antagonisten, die mit der modulierenden Wirkung des Adenosins auf die Freisetzung verschiedener Neuropeptide, metabotroper und ionotroper Glutamat-, Dopamin- und nikotinischer Rezeptoren, die ihrerseits wiederum die Freisetzung von Acetylcholin und Dopamin beeinflussen, sowie mit der Freisetzung von Gamma-Aminobuttersäure (GABA) in Verbindung gebracht werden (J.A. Ribeiro, *Eur. J. Pharmacol.,* **375**, 101-113 (1999)) und potentielle Therapeutika für die Behandlung von Morbus Parkinson darstellen, können daher die Wirkung zerebraler A₁-Agonisten potenzieren (F. Pedata *et al., Ann. N.Y. Acad. Sci.,* **939,** 74-84 (2001)). Letztere stellen potentielle Wirkstoffe für die Therapie von Schlaganfall-Patienten und Patienten mit retinaler Ischämie dar. Ob jedoch Adenosin A_{2A-}Agonisten die Wirkung von Adenosin A₁-Inhibitoren aufheben können, ist derzeit noch nicht bekannt. Allerdings gelten Liganden mit vergleichbarer Bindungsstärke an A₁- und A_{2A-}Rezeptoren als unerwünscht, da die resultierenden pharmakologischen Wirkungen gegeneinander gerichtet sein könnten.

Es ist bekannt, dass Adenosinrezeptor-Antagonisten mit Purin- bzw. Xanthin-Teilstruktur weder ausreichende Affinität noch Selektivität aufwiesen. Jedoch konnten diese Parameter beispielsweise durch Einsatz von 2-Furanyl-Derivaten des Triazolochinazolins, Triazolopyrimidins und Triazolotriazins mit folgenden Strukturformeln signifikant verbessert werden (E. Ongini *et al., Naunyn Schmiedebergs Arch. Pharmacol.,* **359**, 7-10 (1999) und *Farmaco,* **56**, 87-90 (2001):

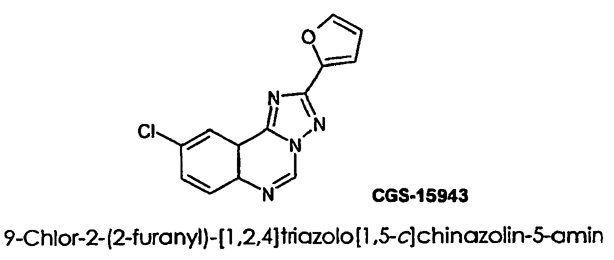

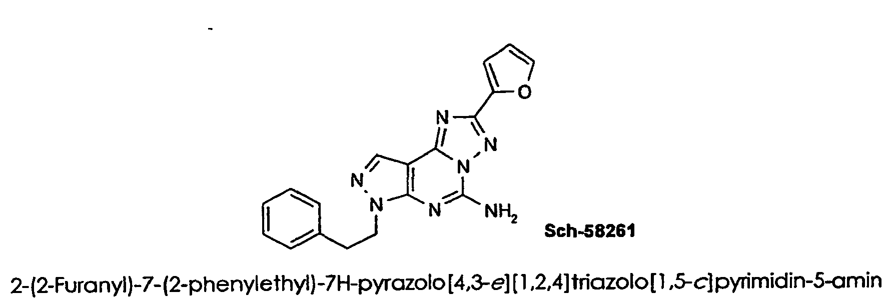

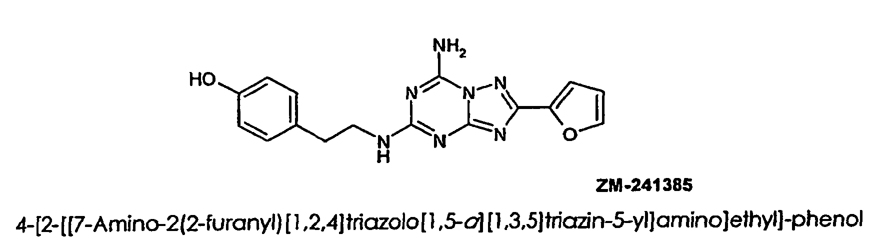

Die angeführten Verbindungen besitzen jedoch unterschiedliche pharmokologische bzw. (physiko)chemische Nachteile. So wurde die Entwicklung der Verbindung **CGS-15943** als Therapeutikum bei ischämischem Schlaganfall wegen mangelnder Selektivität eingestellt. Die Verbindung **Sch-58261** hingegen besitzt zwar eine sehr hohe Rezeptoraffinität und gute Selektivität (*K*iA2A = 2,3 nM; *K*iA1 = 121 nM), die mangelhafte Löslichkeit und schlechte orale Bioverfügbarkeit limitieren jedoch die pharmazeutische Verwendbarkeit. Die Verbindung **ZM-241385,** welche sogar einen Selektivitätsfaktor von ca. 400 aufweist, findet nur als tritiierter Radioligand zur Darstellung von A_{2A}-Rezeptoren im Tierversuch Verwendung.

Neben anderen Triazolo[1,5-*c*]chinazolinen und Triazolo[1,5-*c*]pyrimidinen wurden auch Triazolo-[4,3-*a*]chinoxaline untersucht. Die Tatsache, dass einerseits 4-Amino-6-benzytamino-1-2-dihydro-2-phenyl-1,2,4-triazolo[4,3-*a*]chinoxalin-1-on einen selektiven A_{2A}-Antagonisten darstellt (V. Colotta *et al., Arch. Pharm. Pharm. Med. Chem.,* **332**, 39-41 (1999), und andererseits 8-Chlor-4-*(*cyclohexylamino)-1-(trifluormethyl)[1,2,4]triazolo[4,3-*a*]chinoxalin (CP-68,247) hochselektive antagonistische Wirkung am A₁-Rezeptor entfaltet (IC₅₀-Wert beträgt 28 nM) (R. Sarges *et al., J. Med. Chem.,* **33**, 2240-2254 (1990)), zeigt deutlich, wie nahe diese pharmakologisch gegensätzlichen Wirkungen in dieser Verbindungsklasse beieinander liegen. Für alle diese Verbindungen ist agonistische oder partiell agonistische Wirkung an den jeweiligen Rezeptoren auszuschließen, da eine solche nach dem derzeitigen Stand der Erkenntnisse eine intakte Nukleotidstruktur, jedenfalls aber eine im wesentlichen intakte Riboseeinheit voraussetzt (C.E. Müller und B. Stein, *Curr. Pharm. Design,* **2,** 501-530 (1996) und darin zitierte Literatur).

Ebenso sind aus der Literatur (B. Matuszczak *et al., Arch. Pharm. Pharm. Med. Chem.,* **331,** 163-169 (1998)) Verbindungen mit folgendem Grundkörper bekannt.

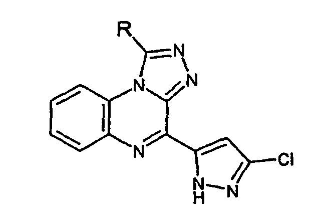

Auch in dieser Substanzklasse zeigte sich eine Abhängigkeit der Subtyp-Affinität vom Substituenten in Position 1. Während das 1-Methyl-Derivat eine höhere Affinität zum Adenosin A_{2A}- als zum A₁-Rezeptor zeigt (A_{2A}: *K*ᵢ = 1,43 µM; A₁: *K*ᵢ *=* 7,85 µM, d.h. der Selektivitätsfaktor beträgt ca. 5), ist bei den übrigen Abkömmlingen eine Bevorzugung des Adenosin A₁-Rezeptors gegeben (für R = 2-Thienylmethyl: *K*_{iA1} = 200 nM). En Vergleich der absoluten Werte der Bindungsaffinität mit denen anderer Adenosinrezeptor-Antagonisten zeigt jedoch, dass die Verbindungen dieses Typs diesbezüglich unterlegen und daher aller Voraussicht nach nicht als Arzneimittel geeignet sind.

### Darstellung der Erfindung

Aufgabe der vorliegenden Erfindung ist es, die Rezeptoraktivitäten der eingangs genannten pyrazoyl-substituierten Triazolochinoxaline sowie deren Analoga in Form von Tetrazolochinoxalinen weiter zu erhöhen.

Erfindungsgemäß werden Triazolochinoxaline der allgemeinen Formel (1) mit Y = CR, insbesondere des 4-(Chlorpyrazolyl)-1-(3-phenylpropyl)-[1,2,4]triazolo[4,3-*a*]chinoxalins sowie Tetrazolochinoxalinen der allgemeinen Formel (2) mit Y = N in 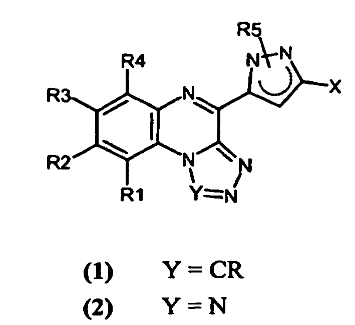 vorgeschlagen, worin R1 bis R4 Wasserstoff, lineare oder verzweigte, gesättigte oder ungesättigte Alkylreste, Cycloalkylreste, welche gegebenenfalls ein oder mehrere Heteroatome aufweisen, Alkoxy-, Hydroxy-, Halogen-, Amino-, Nitro-, Trihalogenmethyl-, Carboxy-, Alkoxycarbonyl- oder Sulfogruppen sind, wobei R1 bis R4 identisch oder verschieden vorliegen können und worin der Substituent R Wasserstoff oder ein linearer oder verzweigtkettiger, gesättigter, und/oder ungesättigter Kohlenstoffrest, ein Cycloalkyl-Rest, ein Phenyl-, Pyridyl-, Thienyl oder Furylrest in unsubstituierter oder ein- bzw. mehrfach substituierter Form ist, wobei der Substituent R entweder direkt oder über eine Alkylengruppe, in welcher ein oder mehrere Kohlenstoffatome durch Heteroatome, wie Sauerstoff, Schwefel oder Stickstoff ersetzt sein können, am Grundkörper gebunden ist, und worin der Substituent R5 Wasserstoff, C₁-C₈ Alkyl, Allyl oder Acyl ist und worin der Rest R6 ein Halogen oder Wasserstoff ist, ausgenommen Verbindungen mit R1 bis R5 gleich Wasserstoff, R6 gleich Chlor und R gleich Methyl, Phenyl, Benzyl, 2-Furyl, 2-Thienyl oder 2-Thienylmethyl.

Die ersten Struktur-Aktivitäts-Beziehungen, die für diese Substanzklasse aufgestellt werden konnten, waren in Übereinstimmung mit einem literaturbeschriebenen Pharmakophormodell für A₁-Antagonisten. (B. Matuszczak *et al., Arch. Pharm. Pharm. Med. Chem.,* **331**, 163-169 (1998)) Diese Resultate ließen vermuten, dass die Struktur-Aktivitäts-Beziehungen klassischer A₁-Liganden auf diese Tricyclen zu übertragen sind. Somit sollte es möglich sein, mit Hilfe bekannter Pharmakophor-Modelle Verbindungen mit verbesserter Rezeptoraffinität sowie -subtypselektivität zu entwickeln.

Überraschenderweise zeigte sich jedoch, dass die Rezeptoraffinitäten weiterer Derivate mit den Pharmakophormodellen nicht mehr in Einklang zu bringen waren. Der Befund, dass nach Entfernen einzelner Strukturbestandteile, die bis zu diesem Zeitpunkt als essentiell für die Ausbildung von Wechselwirkungen des Liganden mit dem Rezeptor angesehen wurden, unveränderte Rezeptoraffinität gegeben war, zeigt, dass eine Analogie hinsichtlich der Bindung am Rezeptor nicht gegeben ist und somit auch Struktur-Affinitäts-Beziehungen aus anderen Substanzklassen nicht auf diese Verbindungen übertragbar ist. Auch literaturbekannte Pharmakophormodelle sind somit auf diese pyrazolylsubstituierten Tricyclen nicht anzuwenden, diese Verbindungsklasse ist vielmehr als vollkommen neue Erfindung anzusehen.

Es war - insbesondere aufgrund der literaturbekannten Pharmakophor- und Rezeptor-Modelle - nicht vorherzusehen, dass die erfindungsgemäßen Verbindungen eine Affinität und Selektivität zu Adenosin-Rezeptoren entfalten, die sie für den Einsatz als Arzneimittel im Sinne von subtypspezifischen Adenosin-Antagonisten in hohem Grade geeignet macht.

Im Rahmen der Erfindung in Betracht gezogene Analoga sind, in gleicher Weise ausgehend von den allgemeinen Formeln (**1**) und (**2**), solche Verbindungen, worin jeweils unabhängig voneinander gilt, dass die Substituenten R1 bis R4 Wasserstoff, lineare oder verzweigte, gesättigte oder ungesättigte Alkylreste, wie beispielsweise Methyl-, Ethyl-, Propyl-, Butyl-, Isobutyl-, Allyl-, Cycloalkylreste, wobei gegebenenfalls ein oder mehrere Kohlenstoffatome durch Stickstoff, Sauerstoff oder Schwefel ersetzt werden können, Alkoxy-, Hydroxy-, Halogen-, Amino-, Nitro-, Trihalogenmethyl-, Carboxy-, Alkoxycarbonyl- oder Sulfogruppen sind, wobei die Substituenten R1 bis R4 identisch oder verschieden sein können.

Der Substituent R kann Wasserstoff oder ein organischer Rest wie ein Alkyl-, Cycloalkyl-, Phenyl-, Pyridyl-, Thienyl- oder Furylrest in unsubstituierter oder ein- bzw. mehrfach substituierter Form sein, der entweder direkt oder über eine Alkylenbrücke, in welcher ein oder mehrere Kohlenstoffatome durch Heteroatome, wie Sauerstoff, Schwefel oder Stickstoff ersetzt sein können, am Grundkörper gebunden ist. Als Substituenten interessieren insbesondere Halogen, unsubstituiertes oder auch substituiertes Alkyl - als Beispiel für eine substituiertes Alkyl interessiert beispielsweise Trifluormethyl -, Alkoxy, Hydroxy, unsubstituiertes oder substituiertes - beispielsweise auch acyliertes - Amino, Alkoxycarbonyl, Carboxyl, Sulfo, Nitro und Cyano verwendet.

Als organischer Rest ist eine lineare oder verzweigtkettige C₁- bis C₆-Alkylgruppe, beispielsweise Methyl, Ethyl, Propyl, Butyl, Isobutyl bevorzugt. Diese Alkylkette kann auch ihrerseits substituiert sein, beispielsweise mit Halogen, Alkoxy, Hydroxy, Amino - unsubstituiert oder mit ein oder zwei Alkyl-, Aryl- oder Acyl-Resten substituiert -, Alkoxycarbonyl, Carboxyl, Sulfo oder Cyano. Des weiteren kann dieser Substituent auch eine oder mehrere Doppel- oder Dreifachbindungen beeinhalten, wie es beispielsweise bei Allylgruppen der Fall ist.

Weiters kann es sich bei dem organischen Rest um einen Cycloalkyl-Substituenten mit der Ringgröße bestehend aus drei bis acht Kohlenstoffatomen handeln. Ein oder mehrere Kohlenstoffatome im Ring können gegen Heteroatome, wie beispielsweise Stickstoff, Sauerstoff, Schwefel ersetzt sein; zudem kann der Ring (beispielsweise ein Piperazin-, *N-*Methylpiperazin-, Morpholin-, Dioxolan-, Dioxan-, Adamantan- oder Noradamantan-Rest) auch weiter substituiert sein.

Der Substituent R5 kann Wasserstoff, eine lineare oder verzweigte Alkyl-, Allyl-, oder Acylgruppe sein. Dieser Substituent befindet sich an einem der Stickstoffatome des PyrazolRinges.

Der Rest R6 kann entweder Halogen, wie Fluor, Chlor, Brom oder aber Wasserstoff sein.

Die Erfindung betrifft weiters Verfahren zur Herstellung der Verbindungen (1) und (2) sowie diese enthaltende Arzneimittel, wie dies gemäß Patentansprüche offenbart ist.

### Wege zur Ausführung der Erfindung

Bezugnehmend auf den Stand der Technik werden im folgenden experimentellen Teil einige Variationsmöglichkeiten erläutert. Diese Beispiele dienen lediglich der Illustration, ohne jedoch die Erfindung auf diesen Umfang einzuschränken.

### I. Verfahren zur Herstellung von Verbindungen gemäß Formel (1) unter Variation der Substituenten R und R1 bis R4:

### I.1 Symmetrisch substituierte Derivate

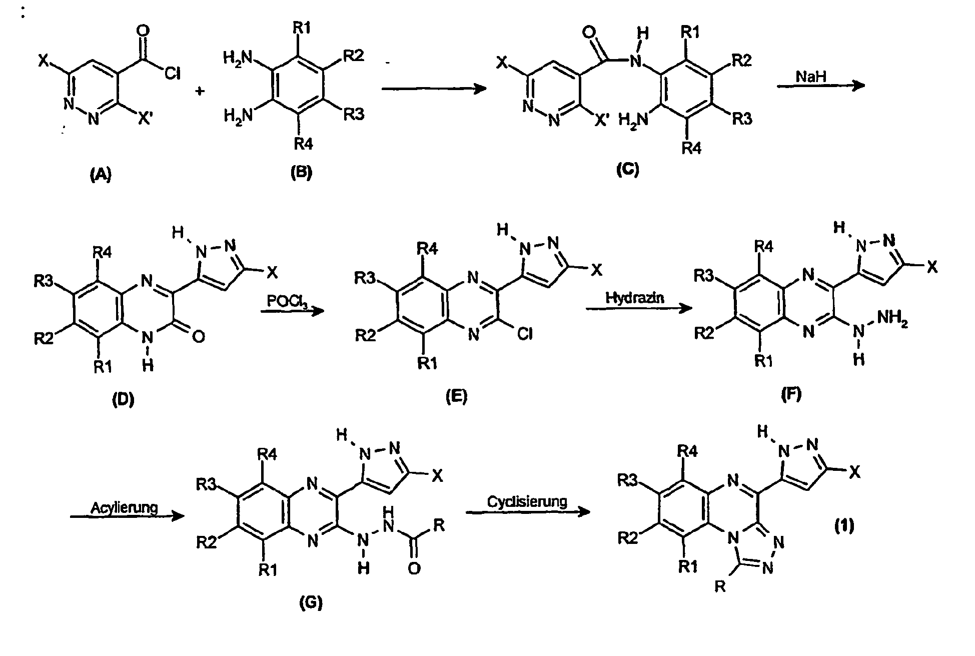

Bei X und X' handelt es sich um den gleichen Rest oder um unterschiedliche Reste der zuvor angeführten Definition mit der Einschränkung X' = Halogen, wobei in der allgemeinen Formel (1) X auch die Bedeutung von R6 hat.

R, R1, R2, R3 und R4 sind entsprechend der zuvor angeführten Beschreibungen zu definieren.

Die dargestellte Synthesesequenz erlaubt nicht nur den Zugang zu Verbindungen, in denen der carbocyclische Teil des Chinoxalins unsubstituiert vorliegt, sondern ermöglicht auch die Darstellung von symmetrisch substituierten Verbindungen des folgenden Typs: 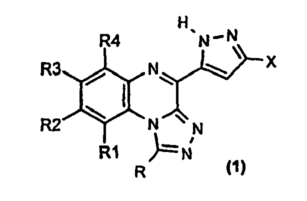
a) R1 bis R4 = idente Substituenten¹
   ¹Substituent bedeutet in diesem Fall R ≠ H;
b) R1 und R4 = idente Substituenten sowie R2 und R3 = H;
c) R2 und R3 = idente Substituenten sowie R1 und R4 = H;
d) R2 und R3 = idente Substituenten sowie R 1 und R4 = idente Substituenten

Die erforderlichen symmetrisch substituierten *ortho*-Phenylendiamine sind käuflich zu erwerben, literaturbeschrieben oder in Analogie zu literaturbeschriebenen Derivaten synthetisch zugänglich. Von besonderer Bedeutung zur Darstellung der substituierten Phenylendiamine sind Nitrierungs- und anschließende Reduktionsreaktionen.

Anhand der Dimethyl-Abkömmlinge soll die Darstellung derartig symmetrisch substituierter Verbindungen (mit X = Cl und R1 = R4 = H sowie R2 = R3 = CH₃) erläutert werden. Die hierfür ausgewählte Substanzklasse bzw. die angeführten Beispiele dienen jedoch lediglich der Illustration, ohne die Erfindung auf deren Umfang zu beschränken.

Im folgenden werden Synthesebeispiele zur Herstellung der im Syntheseschema 11 dargestellten Verbindungen angeführt:

### Synthese von N-(2-Amino-4,5-dimethylphenyl)-3,6-dichlorpyridazin-4-carboxamid* (C-1)

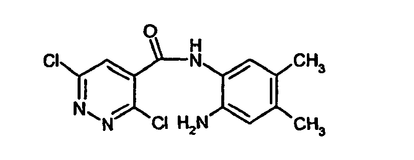

* bereits beschrieben in: M. Banekovich, 'Pyrazolyl-substituierte Chinoxaline: Darstellung neuer potentieller Serotoninrezeptor-Liganden und Untersuchungen zum Fluoreszenz-Verhalten', Diplomarbeit Universität Innsbruck, 1998

Einer Suspension aus 10,440 g (76,65 mmol, 3 Äquivalente) 4,5-Dimethyl-*o*-phenylendiamin und 25,55 mmol Base (beispielsweise Triethylamin, Pyridin, Hünig-Base o.ä.) in 150 mL absolutem Dichlormethan wird bei 0°C unter Stickstoffatmosphäre eine Lösung aus 5,402 g (25,55 mmol) 3,6-Dichlorpyridazin-4-carbonsäurechlorid in 60 ml absolutem Dichlormethan langsam zugetropft. Anschließend wird der Reaktionsansatz bis zur vollständigen Umsetzung des Säurechlorids bei Raumtemperatur gerührt (ca. 16 Stunden; DC (Dünnschichtchromatogramm)-Kontrolle: einige Tropfen des Reaktionsansatzes werden mit verdünnter Salzsäure versetzt, die Lösung wird mit gesättigter Natriumhydrogencarbonat-Lösung neutralisiert und mit Ethylacetat extrahiert, Laufmittel: Ethylacetat). Die entstandenen Kristalle werden abgenutscht, mit Dichlormethan gewaschen und im Exsikkator bis zur Gewichtskonstanz getrocknet.

Das Filtrat wird dreimal mit je 200 ml eisgekühlter verdünnter Natronlauge extrahiert, die wäßrige Phase wird mit Dichlormethan gewaschen und anschließend unter Eiskühlung mit konzentrierter Salzsäure angesäuert. Der resultierende Niederschlag an diacyliertem Produkt wird abgenutscht, die Wasserphase zweimal mit Dichlormethan gewaschen und anschließend mit gesättigter Natriumhydrogencarbonat-Lösung neutralisiert. Die neutrale Lösung wird mit Dichlormethan erschöpfend extrahiert. Die vereinigten organischen Phasen werden mit gesättigter Natriumchlorid-Lösung gewaschen, über Natriumsulfat getrocknet, abfiltriert und zur Trockne eingedampft.

Für die weitere Umsetzung weist das Produkt (hellgelbe Kristalle) der Zusammensetzung C₁₃H₁₂Cl₂N₄O (311,17) (Ausbeute: 90 %) eine ausreichende Reinheit auf.
Schmelztemperatur: ab 198 °C Zersetzung
Elementaranalyse:

| | C | H | N |
|---|---|---|---|
| ber. | 50,18 % | 3,89% | 18,01% |
| gef. | 50,22% | 4,12% | 17,96% |

IR (KBr): 3386, 3183, 3012, 1673 cm⁻¹
¹H-NMR (DMSO-d₆) 9,92 (s, 1 H, NH), 8,47 (s, 1 H, Pyridazin H5), 7,01 (s, 1 H), 6,57 (s, 1 H) (H5, H6), 5,10 (s br, 2H, NH₂), 2,10 (s, 3H, CH₃), 2,08 (s, 3H, CH₃).

### Synthese von 3-[3(5)-Chlor-1H-pyrazol-5(3)-yl]-6,7-dimethylchinoxalin-2(1H)on* (D-1)

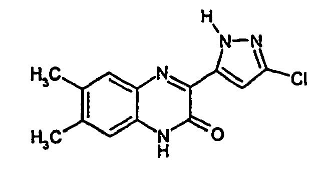

Zu einer Lösung aus 2,000 g (6,44 mmol) *N*-(2-Amino-4,5-dimethylphenyl)-3,6-dichlorpyridazin-4-carboxamid **(C-1)** in 50 ml wasserfreiem Lösungsmittel (beispielsweise *N,N*-Dimethylformamid) werden unter Stickstoffatmosphäre 3 Äquivalente Base (vorzugsweise Natriumhydrid, 60 %ige Dispersion) gegeben und das Reaktionsgemisch wird bis zur vollständigen Umsetzung bei 100 °C gerührt (Reaktionsdauer beträgt ca. 15 Minuten; zur Reaktionskontrolle werden einige Tropfen des Reaktionsansatzes mit verdünnte Salzsäure versetzt und mit Ethylacetat extrahiert, Laufmittel: Ethylacetat; die vollständige Umsetzung ist weiters am Farbumschlag der Reaktionslösung von dunkelrot auf braun erkennbar).

Zur Aufarbeitung wird die Reaktionslösung unter Stickstoffbegasung vorsichtig in verdünnte Salzsäure gegeben. Das dabei kristallin anfallende Produkt wird isoliert und mit Wasser sowie Petrolether gewaschen und anschließend bis zur Gewichtskonstanz getrocknet. Die Reinigung des Rohproduktes erfolgt durch Behandlung einer Lösung der Substanz in Tetrahydrofuran mit Aktivkohle in der Wärme, Filtration und Abdestillation des Lösungsmittels. Das so erhaltene Produkt (gelbe Kristalle) der Zusammensetzung C₁₃H₁₁ClN₄O (274,71) (Ausbeute: 95 %) besitzt für die weitere Umsetzung eine ausreichende Reinheit.
Schmelztemperatur: 328 °C unter Zersetzung
Elementaranalyse:

| | C | H | N |
|---|---|---|---|
| ber. | 56,84 % | 4,04 % | 20,39 % |
| gef. | 56,75 % | 4,34 % | 20,12 % |

IR (KBr): 3311, 1666 cm⁻¹
¹H-NMR (DMSO-d₆) 13,65 (s, 1H, Pyrazol NH), 12,60 (s, 1H, Chinoxalin NH), 7,51 (s, 1H, Chinoxalin CH, 7,15 (d, J = 1,4 Hz, 1 H, Pyrazol-CH), 7,07 (s, 1 H, Chinoxalin CH), 2,29 (s, 3H, CH₃), 2,27 (s, 3H, CH₃).

### Synthese von 2-Chlor-3-[3(5)-chlor-1-H-pyrazol-5(3)-yl]-6,7-dimethylchinoxalin* (E-1)

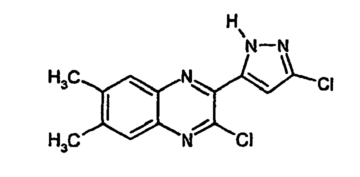

* bereits beschrieben in: M. Banekovich, 'Pyrazolyl-substituierte Chinoxaline: Darstellung neuer potentieller Serotoninrezeptor-Liganden und Untersuchungen zum Fluoreszenz-Verhalten', Diplomarbeit Universität Innsbruck, 1998

Eine Suspension aus 2,601 g (9,47 mmol) des entsprechenden Chinoxalin-2-on-Derivates **(D-1)** wird in einem Gemisch aus 50 mL Phosphoroxychlorid und 5 mL Pyridin bis zur vollstän-digen Umsetzung unter Rückfluß erhitzt (ca. 3 Stunden; DC-Kontrolle: einige Tropfen des Reaktionsansatzes werden mit gesättigter Natriumhydrogencarbonat-Lösung versetzt und mit Ethylacetat extrahiert, Laufmittel: Ether). Nach dem Abkühlen wird das Reaktionsgemisch vorsichtig in Eiswasser gegeben. Um die Kristallisation zu verbessern wird etwas gesättigte Natriumhydrogencarbonat-Lösung zugefügt. Der Niederschlag wird abgetrennt und mit Wasser sowie Petrolether gewaschen und bis zur Gewichtskonstanz im Exsikkator getrocknet. Zur Reinigung wird eine Lösung des Rohproduktes in Tetrahydrofuran mit Aktivkohle versetzt und dieses Gemisch kurz zum Sieden erhitzt, abfiltriert und am Rotavapor bis zur Trockene eingeengt. Das so erhaltene Produkt (beigefarbene Nadeln) der Zusammensetzung C₁₃H₁₀Cl₂N₄ (293,16) weist eine für die weitere Umsetzung ausreichende Reinheit auf (Ausbeute: 1,868 g (67 %)).
Schmelztemperatur: 296-300 °C
Elementaranalyse:

| | C | H | N |
|---|---|---|---|
| ber. | 53,26 % | 3,44 % | 19,11 % |
| gef. | 53,39 % | 3,61 % | 19,23 % |

IR (KBr): 3226 cm⁻¹
¹H-NMR (DMSO-d₆) 13,96 (s, 1 H, Pyrazol NH), 7,89 (s, 1 H, Chinoxalin CH, 7,84 (s, 1 H, Chinoxalin CH), 7,19 (s, 1H, Pyrazol-CH), 2,50 (s, 6H, 2x CH₃).

Anstelle eines Chlorchinoxalins **(E)** können auch andere Derivate zur weiteren Synthese von Verbindungen des Typs **(F)** eingesetzt werden, in welchen das Chloratom durch eine andere Abgangsgruppe, beispielsweise durch Brom, lod oder Mesylat ersetzt ist.

### Anstelle von 3-[3(5)-Chlor- 1H-pyrazol-5(3)-yl]-6,7-dimethyl-2-hydrazinochinoxalin (F-1)

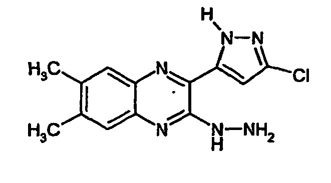

1,061 g (3,62 mmol) des entsprechenden Chlorchinoxalin-Derivates **(E-1)** werden in 20 ml Hydrazinmonohydrat suspendiert und das Gemisch wird bis zur vollständigen Umsetzung unter Rückfluß erhitzt (etwa 2 Stunden; DC-Kontrolle: einige Tropfen des Reaktionsansatzes werden mit Wasser versetzt und mit Ethylacetat extrahiert, Laufmittel: Ethylacetat). Nach dem Abkühlen wird das Reaktionsgemisch in 150 ml Wasser gegeben, zur Vervollständigung der Kristallisation wird die resultierende Suspension einige Stunden bei etwa 4 °C stehen gelassen. Die Kristalle werden anschließend isoliert und mit Wasser sowie Petrolether gewaschen. Nach dem Trocknen bis zur Gewichtskonstanz werden die Kristalle in Tetrahydrofuran gelöst, die Lösung wird mit Aktivkohle versetzt und kurz zum Sieden erhitzt, filtriert und bei vermindertem Druck zur Trockne eingeengt. Das erhaltene Produkt (gelbes Pulver) der Zusammensetzung C₁₃H₁₃ClN₆ (288,74) weist für die weitere Umsetzung eine ausreichende Reinheit auf, analysenreine Verbindung wird durch Umkristallisation aus Tetrahydrofuran erhalten.
Ausbeute: 0,629 g (82 %)
Schmelztemperatur: 281 - 283°C
Elementaranalyse:

| | C | H | N |
|---|---|---|---|
| ber. | 54,08 % | 4,54 % | 29,11 % |
| gef. | 54,38 % | 4,58 % | 29,25 % |

IR (KBr): 3320 cm⁻¹
¹H-NMR (DMSO-d₆): 7,54 (s, 1H), 7,40 (s, 1H) (H5, H8), 7,04 (s, 1H. Pyrazol H4), 2,36 (s, 3H, CH₃), 2,33 (s, 3H, CH₃).

### Allgemeine Arbeitsvorschrift zur Darstellung von 2-Acylhydrazino-3-[3(5)-chlor-1H-pyrazol-5(3)-yl]-6,7-dimethylchinoxalinen des Typs (G-1):

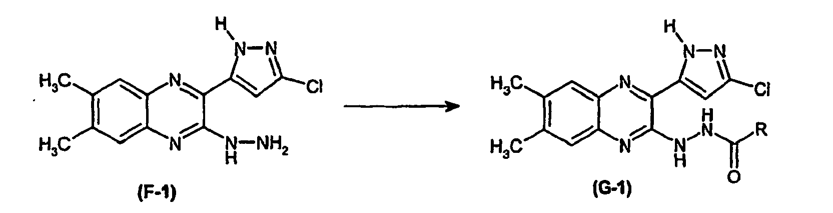

Einer Suspension aus einem Äquivalent Hydrazinochinoxalin (**F-1**) und 1,2 Äquivalenten Base (beispielsweise Triethylamin, Pyridin o.ä.) in einem absolute Lösungsmittel (beispielsweise 1,4-Dioxan, THF, Acetonitril) (10 ml pro mmol Hydrazino-Derivat F-1) wird bei Raumtemperatur eine Lösung von 1,1 Äquivalenten des entsprechenden Acylierungsmittels - beispielsweise Acetylchlorid - in absolutem 1,4-Dioxan (etwa 2 mL pro mmol Acylierungsmittel) langsam zugetropft. Nach vollständiger Zugabe wird der Reaktionsansatz bei Raumtemperatur bis zur vollständigen Umsetzung gerührt (ca. 12-18 Stunden; DC-Kontrolle: einige Tropfen des Reaktionsansatzes werden mit Wasser versetzt und mit Ethylacetat extrahiert, Laufmittel: beispielsweise Ethylacetat). Zur Aufarbeitung wird die Reaktionslösung auf Wasser gegeben und das entstandene Produkt anschließend isoliert: Eine Möglichkeit der Isolierung ist die Abtrennung des resultierenden Niederschlags und anschließendes Waschen des Niederschlages mit Wasser sowie Petrolether. Eine Alternative stellt die Isolierung des Produktes durch Extraktion mit einem geeigneten organischen Lösungsmittel dar, in diesem Fall wird die Lösung des acylierten Produktes anschließend mit Wasser und gesättigter Natriumchlorid-Lösung gewaschen sowie getrocknet, abschließend wird das organische Lösungsmittel destillativ entfernt.

Zur Reinigung des jeweiligen acylierten Derivates wird das entsprechende, bis zur Gewichtskonstanz getrocknete Produkt aus einem geeigneten Lösungsmittel umkristallisiert. Sofem erforderlich, wird das Rohprodukt zuvor in einem geeigneten Lösungsmittel - beispielsweise Tetrahydrofuran - gelöst, mit Aktivkohle versetzt, kurz aufgekocht, abfiltriert und im Vakuum zur Trockne eingeengt.

### Synthese von 2-Acetylhydrazino-3-[3(5)-chlor-1H-pyrazol-5(3)-yl]-6,7-dimethylchinoxalin (G-1A)

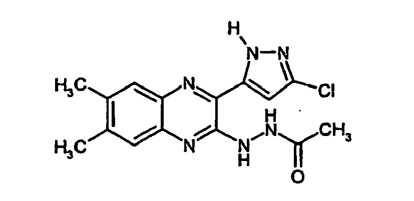

Die Herstellung der Verbindung G-1 A erfolgt gemäß der allgemeinen Arbeitsvorschrift zur Herstellung von Verbindungen des Typs G-1 unter Verwendung folgender Reaktionsparameter bzw. Reaktionsmittel:
Acylierungsmittel: Acetylchlorid
Reaktionszeit: 14 Stunden
Aussehen: gelbe Kristalle
Ausbeute: 61 %
Summenformel: C₁₅H₁₅ClN₆O (330,78)
Schmelztemperatur: 316 - 320 °C (aus Ethylacetat-Tetrahydrofuran (1:1))
Elementaranalyse:

| | C | H | N |
|---|---|---|---|
| ber. | 54,47 % | 4,57 % | 25,41 % |
| gef. | 54,39 % | 4,59 % | 25,15 % |

IR (KBr): 3261, 1500 cm⁻¹
¹H-NMR (DMSO-d₆ + D₂O): 7,66 (s, 1 H), 7,47 (s, 1 H) (H5, H8), 7,09 (s, 1 H, Pyrazol H4), 2,38 (s, 3H, CH₃), 2,37 (s, 3H, CH₃), 1,98 (s, 3H, CH₃).

### Allgemeine Arbeitsvorschrift zur Synthese von 1-substituierten 4-[3(5)-Chlor-1H-pyrazol-5(3)-yl]-7,8-dimethyl-[1,2,4]triazolo [4,3-a]chinoxalinen gemäß der allgemeinen Formel (1):

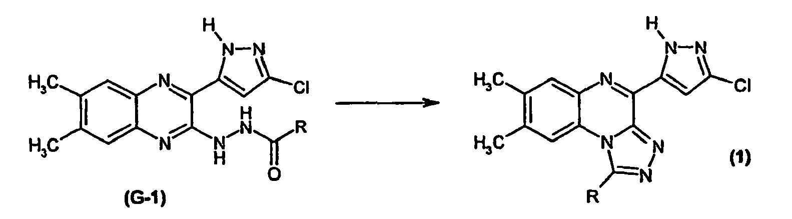

Eine Suspension Säurehydrazid-Derivat (**G-1**) in einem Gemisch aus 1,2-Dichlorethan (20 ml pro mmol) und Phosphoroxychlorid (8 ml pro mmol) wird bis zur vollständigen Umsetzung unter Rückfluß erhitzt (etwa 2 Stunden; DC-Kontrolle: einige Tropfen des Reaktionsansatzes werden mit Wasser versetzt und mit Ethylacetat extrahiert, Laufmittel: geeignetes organisches Lösungsmittel bzw. -gemisch, beispielsweise Ethylacetat).

Zur Aufarbeitung wird der abgekühlte Reaktionsansatz vorsichtig auf Eiswasser gegeben, das resultierende kristalline Produkt wird isoliert und mit Wasser sowie Petrolether gewaschen. Nach dem Trocknen wird das Produkt in Tetrahydrofuran gelöst, mit Aktivkohle versetzt und das Gemisch für einige Minuten unter Rückfluß erhitzt. Das Filtrat wird im Vakuum zur Trockne eingeengt und die zurückbleibende Substanz anschließend aus einem geeigneten Lösungsmittel umkristallisiert .

### Synthese von 4-[3(5)-Chlor-1H-pyrazol-5(3)-yl]-1,7,8-trimethyl-[1,2,4]triazolo[4,3-a]chinoxalin (I.1.A):

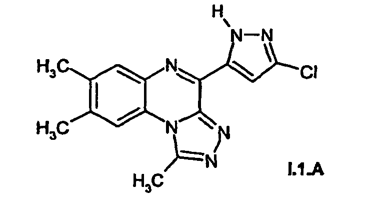

Die Synthese dieser Verbindung erfolgt mittels vorgenannter, allgemeiner Arbeitsvorschrift, wobei die Reaktionszeit zwei Stunden beträgt.
Aussehen: hellgelbes Pulver
Ausbeute: 57%
Summenformel: C₁₅H₁₃ClN₆ (312,76)
Schmeiztemperatur 278-281°C (aus Ethylacetat)
Elementaranalyse: (bezogen auf C₁₅H₁₃ClN₆ x 0,1 H₂O x 0,1 Ethylacetat)

| | C | H | N |
|---|---|---|---|
| ber. | 57,20 % | 4,36 % | 25,99 % |
| gef. | 57,00 % | 4,27 % | 25,98 % |

IR (KBr): 3431 cm⁻¹
¹H-NMR (DMSO-d₆): 14,10 (s, 1H, NH), 8,06 (s, 1H), 7,80 (s, 1H) (H6, H9), 7,54 (s, 1H, Pyrazol H4), 3,11 (s, 3H, CH₃), 2,45 (s, 3H, CH₃), 2,39 (s, 3H, CH₃).

### I.2 Unsymmetrisch substituierte Derivate

Da die Umsetzung unsymmetrisch substituierter *ortho*-Phenylendiamine mit 3,6-Dihalogenpyridazin-4-carbonsäurechlorid im allgemeinen zur Bildung eines Isomerengemisches führt und eine Trennung der isomeren Verbindungen üblicherweise mit sehr groBem Zeit- und Materialaufwand verbunden ist, erlaubt das zuvor beschriebene Verfahren keinen besonders effektiven Zugang zu unsymmetrisch substituierten Triazolochinoxalin-Derivaten. Die nachfolgend beschriebene alternative Synthesestrategie ermöglichst es hingegen, isomerenreine Produkte zu erhalten und stellt damit eine deutliche Verbesserung gegenüber der derzeitigen Vorgangsweise dar.

Dieses erfindungsgemäße Verfahren zeichnet sich dadurch aus, daß primär eine *N*-Acylierung eines *ortho*-Nitroanilin-Derivates durchgeführt wird und erst im nachfolgenden Schritt die zweite Aminogruppe gebildet wird. Letzteres erfolgt durch Reduktion der Nitro-gruppe. Die weiteren Reaktionsschritte entsprechen jenen zur Herstellung von symmetrisch substituierten Derivaten:

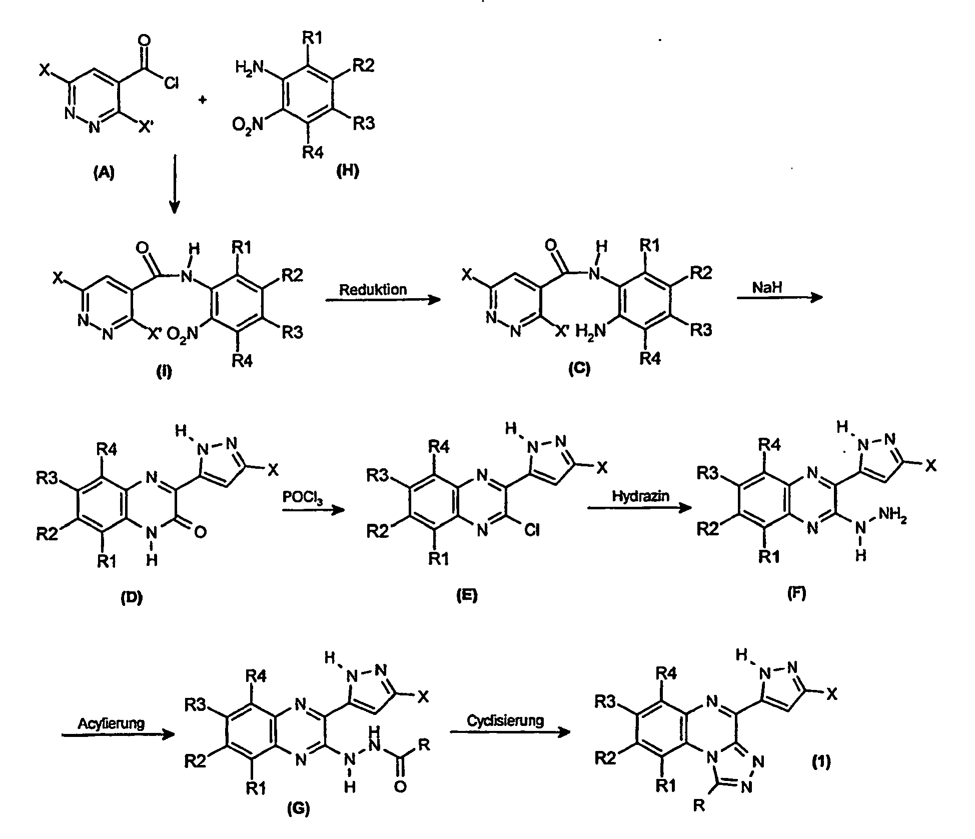

Bei X und X' handelt es sich um den gleichen Rest oder um unterschiedliche Reste der zuvor angeführten Definition mit der Einschränkung X' = Halogen, wobei X die Bedeutung von R6 hat.

R, R1, R2, R3 und R4 sind entsprechend der zuvor angeführten Beschreibungen zu definieren.

Die erforderlichen substituierten 2-Nitroaniline sind käuflich zu erwerben, literaturbeschrieben oder in Analogie zu literaturbeschriebenen Derivaten synthetisch zugänglich. Von besonderer Bedeutung sind hier Nitrierungsreaktionen.
Am Beispiel des Monomethoxy-Derivates wird diese Synthesestrategie im folgenden näher beschrieben, die Erfindung soll jedoch nicht auf diese beschränkt sein.

Anstelle von ortho-Nitroanilin-Derivaten können auch Abkömmlinge von substituierten ortho-Phenylendiaminen eingesetzt werden, sofern einer der beiden Stickstoffatome eine Schutzgruppe trägt und somit einerseits sichergestellt ist, daß bei der Acylierung lediglich ein Isomer resultiert, andererseits sollte die Schutzgruppe unter äußerst milden Reaktionsbedingungen abspaltbar sein. Geeignete Schutzgruppen sind aus der Literatur bekannt; hier können beispielsweise *N,O*-Acetale, *N*-Benzyl-, *N*-Benzyloxycarbonyl-, *N*-*tert*.-Butyl*-*, *N*-[Di(p-methoxyphenyl)methyl]-, *N*-(2,4-Dimethoxybenzyl)-, *N*-[9-Phenylfluoren-9-yl]-, *N*-Silyl-Abkömmlinge oder auch Imine eingesetzt werden.

### Synthese von N-(4-Methoxy-2-nitrophenyl)-3,6-dichlor-4-pyridazincarboxamid (J-1)

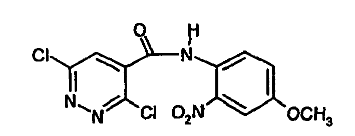

5,00 g (32,86 mmol) 4-Methoxy-2-nitroanilin werden in 60 ml absolutem Dichlormethan gelöst und mit 3,99 g (39,43 mmol, 1,2 Äquivalente) Triethylamin versetzt. Nach dem Enleiten von Stickstoff wird unter Eiskühlung und ständigem Rühren 7,30 g (34,51 mmol, 1,05 Äquivalente) eine Lösung von 3,6-Dichlorpyridazin-4-corbonsäurechlorid in einigen ml absolutem Dichlormethan langsam zugetropft. Anschließend wird der Reaktionsansatz auf Raumtemperatur gebracht und bis zur vollständigen Umsetzung weitergerührt (ca. 12 Stunden; DC-Kontrolle: Laufmittel: Dichlormethan/Ethylacetat = 9/1). Zur Aufarbeitung wird das Reaktionsgemisch mit 50 ml Dichlormethan verdünnt, und die organische Phase wird mehrmals mit je 100 ml verdünnter Salzsäure gewaschen. Ein Teil des Produktes fällt hierbei in kristalliner Form (DC-rein) an und kann abgenutscht werden.

Die vereinigten organischen Phasen werden mit Wasser und gesättigter Natriumchlorid-Lösung gewaschen, über wasserfreiem Natriumsulfat getrocknet, abfiltriert und am Rotavapor bis zur Trockne eingeengt. Der Rückstand wird anschließend aus Ethylacetat umkristallisiert.
- Aussehen:: nahezu farbloses Pulvers
- Ausbeute: .: 8,36 g (74 %)
- Schmelztemperatur:: 198 - 201 °C
- Summenformel:: C₁₂H₈Cl₂NaO₄, (343,13)
- IR (KBr):: 1720 cm⁻¹
- ¹H-NMR (DMSO-d₆):: 8,38 (s, 1H, Pyridazin H), 7,99 (d, J = 8,8 Hz, 1H, H6), 7,74 (d, J = 2,9 Hz, 1 H, H3), 7,51 (dd, J = 8,8 Hz, J = 2,9 Hz, 1 H, H5), 3,88 (s, 3H, OCH₃)

### Reduktion von N-(4-Methoxy-2-nitrophenyl)-3,6-dichlor-4-pyridazincarboxamids (J-1)

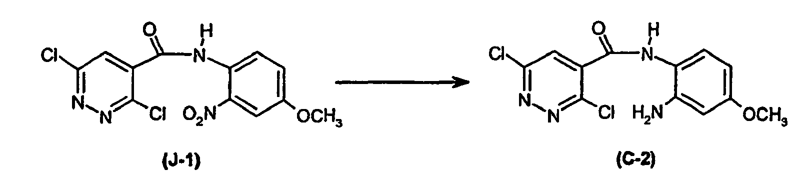

2,000 g (5,83 mmol) *N*-(4-Methoxy-2-nitrophenyl)-3,6-dichlor-4-pyridazincarboxamid (**J-1**) werden in 50 ml Tetrahydrofuran gelöst. Zu dieser Lösung werden unter Stickstoffatmosphäre 3,676 g (58,29 mmol, 10 Äquivalente) Ammoniumformiat und 0,864 g Palladium auf Aktivkohle (10 %-ig) gegeben, wonach der Reaktionsansatz bis zur vollständigen Umsetzung bei Raumtemperatur gerührt wird (ca. 18 Stunden; DC-Kontrolle; Laufmittel: Dichlormethan/Ethylacetat = 30/1).

Zur Aufarbeitung des Reaktionsansatzes wird der Katalysator abfiltriert und das Filtrat zur Trockne eingeengt. Der Rückstand wird mit Wasser versetzt und das Produkt durch erschöpfende Extraktion mit Ethylacetat isoliert. Die gesammelten organischen Phasen werden mit Wasser sowie gesättigter Natriumchlorid-Lösung gewaschen und über Natriumsulfat getrocknet. Nach Abdestillation des Lösungsmittels verbleiben 1,403 g (77 %) eines gelben Pulvers der Zusammensetzung C₁₂H₁₀Cl₂N₄O₂, welches ohne weitere Reinigung weiter umgesetzt werden kann.
- Schmelztemperatur:: 150 - 155 °C
- ¹H-NMR (DMSO-d₆):: 9,86 (s, 1 H, NH), 8,49 (s, 1 H, Pyridazin H5), 7,10 (d, J = 8,8 Hz, 1 H), 6,34 (d, J = 2,6 Hz, 1 H, H3), 6,19 (dd, J = 8,8 Hz, J = 2,6 Hz, 1H, H5), 5,08 (s, 2H, NH₂), 3,69 (s, 3H, OCH₃)

### Synthese von 3-(3-Chlor-1H-pyrazol-5-yl)- 6-methoxychinoxalin-2(1H)-on (D-2)

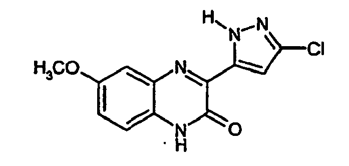

Eine Lösung von 0,695 g (2,22 mmol) *N*-(2-Amino-4-methoxyphenyl)-3,6-dichlor-4-pyridazincarboxamid (**C-2**) in 25 ml absolutem *N,N*-Dimethylformamid wird unter Stickstoffatmosphäre mit 0,266 g (6,66 mmol, 3 Äquivalente) Natriumhydrid versetzt. Das Reaktionsgemisch wird bis zur vollständigen Umsetzung bei 100°C gerührt (Reaktionsdauer: ca. 15 Minuten; zur Reaktionskontrolle werden einige Tropfen des Reaktionsansatzes mit verdünnter Salzsäure versetzt und mit Ethylacetat extrahiert, DC-Kontrolle des Extraktes Laufmittel: Ethylacetat; die vollständige Umsetzung ist weiters am Farbumschlag der Reaktionslösung von dunkelrot auf braun erkennbar).

Zur Aufarbeitung wird die Reaktionslösung vorsichtig unter Stickstoffatmosphäre in 75 ml verdünnte Salzsäure gegeben, wonach die resultierende Suspension einige Stunden bei 4 °C stehen gelassen wird. Das kristallin ausfallende Produkt wird abgenutscht und mit Wasser sowie Petrolether gewaschen. Der bis zur Gewichtskonstanz getrocknete Rückstand wird zur Reinigung in Tetrahydrofuran gelöst, die Lösung wird mit Aktivkohle versetzt und das Gemisch kurz unter Rückfluß erhitzt, abfiltriert und am Rotavapor zur Trockne eingeengt. Dieses Produkt weist für die weitere Umsetzung eine ausreichende Reinheit auf. Die analysenreine Substanz der Zusammensetzung C₁₂H₉ClN₄O₂ (276,68) wird durch Umkristallisation als gelbes Pulver aus Ethylacetat erhalten;
Ausbeute: 0,490 g (80 %).
Schmelztemperatur: 328 - 330 °C
Elementaranalyse: (bezogen auf C₁₂H₉ClN₄O₂ x 0,1 Ethylacetat)

| | C | H | N |
|---|---|---|---|
| ber. | 52,17 % | 3,46 % | 19,62 % |
| gef. | 52,47 % | 3,62 % | 19,49 % |

IR (KBr): 3317, 2811, 1663 (C=O) cm⁻¹
¹H-NMR (DMSO-d₆): 13,74 (s, 1 H, Pyrazol NH), 12,71 (s, 1 H, Chinoxalin NH), 7,33 - 7,20 (m, 4H, H5, H7, H8, Pyrazol-H4), 3,83 (s, 3H, OCH₃)

### Synthese von 2-Chlor-3-(3-chlor-1H-pyrazol-5-yl)-6-methoxychinoxalin (E-2)

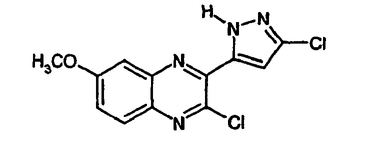

0,910 g (3,29 mmol) des entsprechenden Chinoxalin-2-on-Derivates (**D-2**) werden unter Eiskühlung in 15 ml Phosphoroxychlorid gelöst und mit 1,5 ml Pyridin versetzt, das Reaktionsgemisch wird anschließend bis zur vollständigen Umsetzung unter Rückfluß erhitzt (etwa 3 Stunden; DC-Kontrolle: einige Tropfen des Reaktionsansatzes werden mit gesättigter Natriumhydrogencarbonat-Lösung versetzt und mit Ethylacetat extrahiert, Laufmittel: Ether). Zur Aufarbeitung wird der Reaktionsansatz zunächst auf 0 °C abgekühlt, anschließend vorsichtig in Wasser gegeben und zur Verbesserung der Kristallisation mit gesättigter Natriumhydrogencarbonat-Lösung neutralisiert. Nach mehrstündigem Stehen bei 4 °C wird der Niederschlag abgetrennt. Die analysenreine Verbindung der Zusammensetzung C₁₂H₈Cl₂N₄O (295,13) wird durch Umkristallisation aus Ethylacetat in Form gelber Nadeln erhalten:
Ausbeute: 0,660 g (68 %).
Schmelztemperatur: 243 - 246 °C
Elementaranalyse:

| | | C | H | N |
|---|---|---|---|---|
| | ber. | 48,84 % | 2,73 % | 18,98 % |
| | gef. | 49,04 % | 2,98 % | 18,76 % |

¹H-NMR (DM-SO-d₆): 13,99 (s, 1 H, Pyrazol NH), 7,98 (d, J = 9,0 Hz, 1 H, H8), 7,58 (dd, J = 9,0 Hz, J = 2,9 Hz, 1 H, H7), 7,45 (d, J = 2,9 Hz, 1 H, H5), 7,22 (s, 1H, Pyrazol-H4), 3,98 (s, 3H, OCH₃)

### Synthese von 3-[3(5)-Chlor-1H-pyrazol-5(3)-yl]-2-hydrazino-6-methoxychinoxalin (F-2)

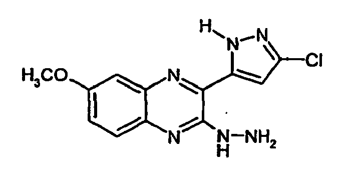

Eine Suspension von 0,670 g (2,27 mmol) 2-Chlor-3-(3-chlor-1*H*-pyrazol-5-yl)chinoxalins (**E-2**) wird in 15 ml Hydrozinmonohydrat suspendiert, wonach das Reaktionsgemisch bis zur vollständigen Umsetzung unter Rückfluß erhitzt wird (Reaktionsdauer: 2 Stunden; DC-Kontrolle: einige Tropfen des Reaktionsansatzes werden mit Wasser versetzt und mit Ethylacetat extrahiert, Laufmittel: Ether). Zur Aufarbeitung wird der Reaktionsansatz auf 75 ml Wasser gegeben und einige Stunden bei etwa 4°C stehen gelassen. Der entstandene Niederschlag wird isoliert, mit Wasser und Petrolether gewaschen und anschließend im Vakuum bis zur Gewichtskonstanz getrocknet. Zur Reinigung wird das Rohprodukt in Tetrahydrofuran gelöst, mit Aktivkohle versetzt und dieses Gemisch einige Minuten unter Rückfluß erwärmt. Nach dem Entfernen der Aktivkohle wird das Lösungsmittel abdestilliert, der resultierende helle Rückstand wird anschließend in Ether suspendiert, der Feststoff wird isoliert und mit Ether gewaschen. Das so erhaltene Produkt der Zusammensetzung C₁₂H₁₁ClN₆O (290,71) weist für die anschließende Umsetzung ausreichende Reinheit auf. Für analytische Zwecke erfolgt eine Umkristallisation aus Ethylacetat, welche ein beige-grünliches Pulver in 60 %iger Ausbeute (0.396 g) liefert.
Schmelztemperatur: 219 - 222 °C
Elementaranalyse: (bezogen auf C₁₂H₁₁ClN₆O x 0,2 Ethylacetat)

| | C | H | N |
|---|---|---|---|
| ber. | 49,86 | 4,12 | 27,26 |
| gef. | 50,16 | 4,16 | 27,01 |

¹H-NMR (DMSO-d₆ + D₂O): 7,58 (d, J = 9,0 Hz, 1 H, H8), 7,28-7,25 (m, 2H, H5, H7), 7,08 (s, 1H, Pyrazol-H4), 3,86 (s, 3H, OCH₃)

### Einführen eines Acylrestes in die Verbindung F-2 zur Herstellung von Hydraziden des Typs G-2

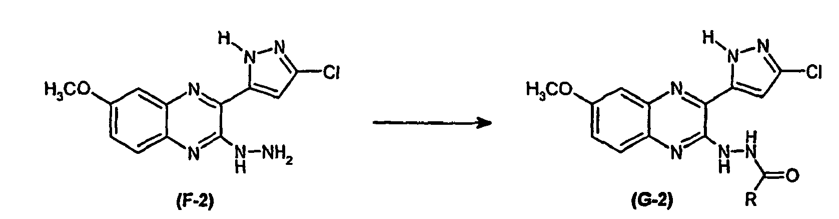

Die Synthesevorschrift entspricht der allgemeinen Arbeitsvorschrift zur Darstellung von Verbindungen des Typs **G-1**.

Die beispielhafte Erläuterung erfolgt am folgenden Synthesebeispiel:

### Synthese von 3-[3(5)-Chlor-11H-Pyrazol-5(3)-yl]-6-methoxy-2-(2-thienylacetyl)-hydrazino chinoxalin (G-2A)

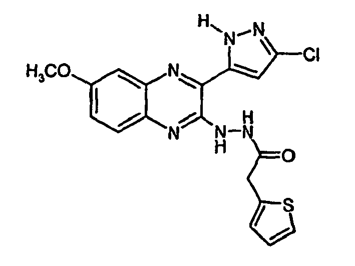

0,240 g (0,63 mmol) Hydrazinochinoxalin **F-2** werden in 30 ml absolutem 1,4-Dioxan suspendiert und mit 0,100 g (0,99 mmol, 1,2 Äquivalente) Triethylamin versetzt. Unter Schutzgasatmosphäre wird bei Raumtemperatur langsam eine Lösung von 0,146 g (0,91 mmol, 1,1 Äquivalente) 2-Thienylacetylchlorid in 5 ml absolutem 1,4-Dioxan zugetropft. Die Reaktionslösung wird anschließend bis zur vollständigen Umsetzung bei Raumtemperatur gerührt (ca. 14 Stunden; zur Reaktionskontrolle werden einige Tropfen des Reaktionsansatzes mit verdünnter Salzsäure versetzt und mit Ethylacetat extrahiert, Laufmittel: Ethylacetat). Zur Aufarbeitung wird der Reaktionsansatz in 75 ml Wasser gegeben und das Gemisch zur Verbesserung der Kristallisation mit einigen Milliliter verdünnter Salzsäure versetzt.

Nach mehrstündigem Stehen bei etwa 4 °C wird das kristalline Produkt isoliert und mit Wasser sowie Petrolether gewaschen. Zur Reinigung wird die getrocknete Substanz in Tetrahydrofuran gelöst und mit Aktivkohle versetzt. Dieses Gemisch wird kurz unter Rückfluß erhitzt, anschließend wird filtriert und das Lösungsmittel mittels Destillation entfernt. Das auf diese Weise in 57 %iger Ausbeute erhaltene Produkt (gelblich-beiges Pulver) der Zusammensetzung C₁₈H₁₅ClN₆O₂S (414,88) weist eine für die weitere Umsetzung ausreichende Reinheit auf.

### Synthese von 1-substituierten 4-[3(5)-Chlor-1H-pyrazol-5(3)-yl]-7-methoxy-[1,2,4]triazolo [4,3-a]-chinoxalinen des Typs (1):

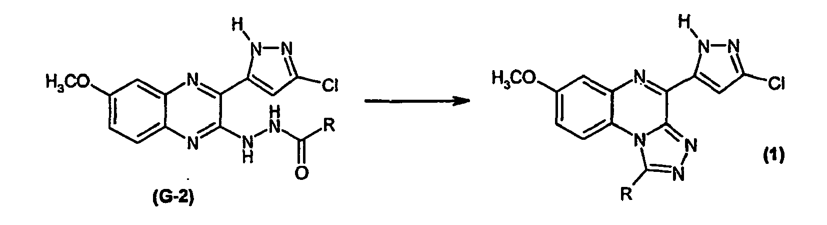

Die Synthese entspricht der Arbeitsvorschrift zur Cyclisierung von Verbindungen des Typs **G-1** und wird an folgendem Synthesebeispiel näher erläutert.

### Synthese von 4-[3(5)-Chlor-1H-pyrazol-5(3)-yl]-7-methoxy-1-(2-thienylmethyl)-[1,2,4]triazolo[4,3-a]-chinoxalin (I.2.A):

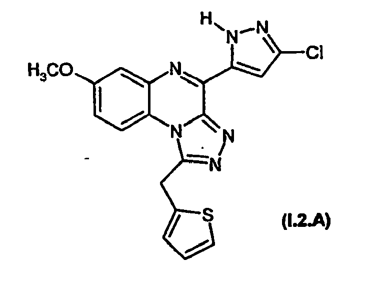

0,195 g (0,47 mmol) des Säurehydrazids **G-2A** werden in einem Gemisch aus 5 ml Phosphoroxychlorid und etwa 15 ml eines inerten Lösungsmittels (z.B. 1,2-Dichlorethan oder Acetonitril) bis zur vollständigen Umsetzung unter Rückfluß erhitzt (ca. 2 Stunden; DC-Kontrolle: einige Tropfen des Reaktionsansatzes werden mit Wasser versetzt, die Lösung wird mit gesättigter Natriumhydrogencarbonat-Lösung neutralisiert und mit Ethylacetat extrahiert, Laufmittel: Ethylacetat).

Nach dem Abkühlen wird das Reaktionsgemisch vorsichtig auf 100 ml Eiswasser gegeben, die resultierenden Kristalle werden isoliert, mit Wasser und Petrolether gewaschen und getrocknet. Das wäßrige Filtrat wird dreimal mit je 100 ml Ethylacetat extrahiert, die organische Phase wird mit verdünnter Natronlauge, Wasser sowie gesättigter Natriumchlorid-Lösung gewaschen und über Natriumsulfat getrocknet. Der nach Abdestillation des Lösungsmittels verbleibende Rückstand wird zur Reinigung in Tetrahydrofuran gelöst, mit Aktivkohle versetzt und kurz zum Sieden erhitzt. Das nach Entfernen der Aktivkohle erhaltene Filtrat wird zur Trockne eingedampft. Das erhaltene Rohprodukt wird mittels Säulenchromatographie (stationäre Phase: Kieselgel; mobile Phase: Ethylacetat) sowie Umkristallisation aus Ethylacetat gereinigt.
Aussehen: gelblich-braunes Pulver
Ausbeute: 0,170 g (91 %)
Summenformel: C₁₈H₁₃ClN₆OS (396,86)
Schmebtemperatur: 203-205 °C
Elementaranalyse: (bezogen auf C₁₈H₁₃ClN₆OS × 0.4THF)

| | C | H | N |
|---|---|---|---|
| ber. | 54,48 % | 3,78 % | 19,45 % |
| gef. | 54,45 % | 3,70 % | 19,39 % |

¹H-NMR (CDCl₃): 7,92 (d, J = 9,0 Hz, 1 H, H9), 7,90 (s, 1 H, Pyrazol-4), 7,76 (d, J = 3,0 Hz, 1 H, H6), 7,23 - 7,20 (m, 1 H), 6,94 - 6,00 (m, 1 H), 6,79 - 6,78 (m, 1 H) (Thiophen-H3, -H4, -H5), 7,13 (dd, J = 9,0 Hz, J = 3,0 Hz, 1 H, H8), 5,08 (s, 2H, CH₂), 3,91 (s, 3H, OCH₃)

Je nach Substitutionsmuster lassen sich Verbindungen mit Substituenten am Chinoxalin-Ring weiter derivatisieren. Diese Derivatisierungen umfassen beispielsweise die dem Fachmann bekannten Verfahren, wie die Acylierung, Alkylierung, Nitrierung, Reduktion, Hydroxylierung, Etherspaltung etc.

Unsymmetrisch substituierte Derivate können, sofern der vorliegende Substituent bzw. die Substituenten einen Einfluß auf die Acylierungsposition besitzt/besitzen, zudem auch ausgehend von substituierten *ortho*-Phenylendiaminen dargestellt werden. Dieses ist beispielsweise beim Vorliegen elektronenabziehender Substituenten gegeben. Ausgehend von 4-Nitrophenylendiamin soll diese Variante der Darstellung unsymmetrisch substituierter Verbindungen (beispielsweise mit X, X' = Cl und R2 = NO₂, R1 = R3 = R4 = H) nachfolgend illustriert werden, ohne damit aber die Erfindung auf diesen Umfang zu beschränken. Die weiteren Reaktionsschritte werden wie zuvor beschrieben durchgeführt.

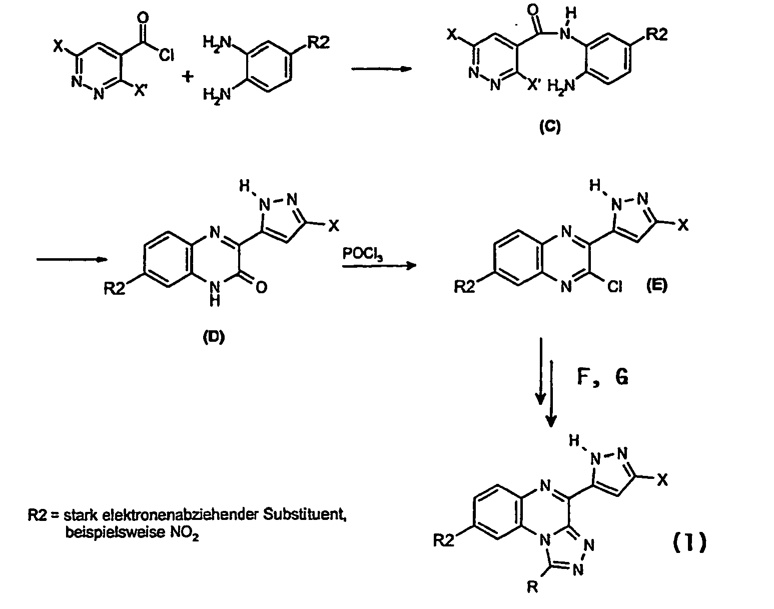

Eine analoge Vorgangsweise kann beim Vorliegen von Substituenten, welche einen gegenteiligen Effekt besitzen, d.h. Aktivierung der Aminogruppierung, angewendet werden. Beide Varianten sind auch beim Ensatz mehrfach substituierter Phenylendiamine erfolgreich.

Je nach Substitutionsmuster können nachträglich weitere Modifikationen vorgenommen werden. Ausgehend von der Nitrogruppierung ist insbesondere die Reduktion zur Aminogruppe von besonderer Bedeutung. Diese Funktion kann weiterführend derivatisiert - z.B. acyliert oder alkyliert werden, auch über ein Diazoniumsalz kann diese Funktion modifiziert werden.

Das vorgenannte Synthesesystem wird anhand folgender Synthesebeispiele näher erläutert:

### Synthese von N-(2-Amino-5-nitrophenyl)-3,6-dichlorpyridazin-4-carboxamid (C-3)

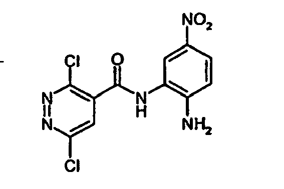

Zu einem Gemisch äquimolarer Mengen 4-Nitrophenylendiamin und Base (beispielsweise Pyridin, Triethylamin, Hünig-Base) in absolutem Lösungsmittel (z.B. Tetrahydrofuran, Dichlormethan, 1,4-Dioxan, DMF o.ä.) wird unter Stickstoffatmosphäre und Eiskühlung die Lösung eines Äquivalents 3,6-Dichlor-4-pyridazincarbonsäurechlorid in absolutem Lösungsmittel langsam getropft. Der Reaktionsansatz wird bei Raumtemperatur bzw. erhöhter Temperatur, d.h. bis zur Siedehitze des eingesetzten Lösungsmittels, bis zur vollständigen Umsetzung gerührt. Zur Beschleunigung kann der Reaktionsansatz auch für einige Minuten im Ultraschall behandelt werden (DC-Kontrolle: einige Tropfen des Reaktionsansatzes werden mit verdünnter Salzsäure versetzt, die Lösung wird mit gesättigter Natriumhydrogencarbonat-Lösung neutralisiert und mit Ethylacetat extrahiert, Laufmittel: geeignetes organisches Lösungsmittel, beispielsweite Ethylacetat). Anschließend wird das Reaktionsprodukt isoliert, was durch Abtrennung der entstandenen Kristalle oder durch Extraktion mit einem organischen Lösungsmittel erfolgt. Reine Kristalle werden durch Umkristallisation erhalten.
- Aussehen:: gelblich-orange Kristalle
- Schmelzpunkt:: 244 °C (aus 1,4-Dioxan)
- Ausbeute:: 89 %
- Summenformel:: C₁₁H₇Cl₂N₅O₃ (328,12)
- IR (KBr):: 3448 ; 3259 (NH₂); 1673 (C=O)
- ¹H-NMR (DMSO-d₆):: 10,41 (s (br), 1 H, NH, mit D₂O austauschbar); 8,53 (s, 1 H, Pyridazin-H5); 8,27 (d, J = 2,6 Hz, 1 H, Phenyl-H3); 7,94 (dd, J = 9,2 Hz; J = 2,6 Hz, 1 H, Phenyl-H5); 6,83 (d, J = 9,2 Hz, 1 H, Phenyl-H6); 6,68 (s, 2 H, NH₂, mit D₂O austauschbar)

### Synthese von 3-(5-Chlor-2H-pyrazol-3-yl)-7-nitro-H-chinoxalin-2-on (D-3)

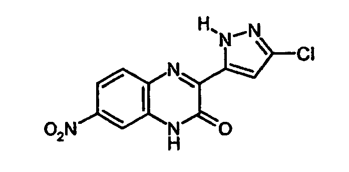

Zu einer Lösung aus einem Äquivalent *N*-(2-Amino-5-nitrophenyl)-3,6-dichlorpyridazin-4-carboxamid (**C-3**) in einem wasserfreiem Lösungsmittel (beispielsweise *N,N*-Dimethylformamid) werden unter Stickstoffatmosphäre 3 Äquivalente Base (vorzugsweise 60 %-ige Natriumhydrid-Dispersion) gegeben, wonach das Reaktionsgemisch bis zur vollständigen Umsetzung bei 100 °C gerührt wird (Reaktionsdauer: ca. 15 Minuten: zur Reaktionskontrolle werden einige Tropfen des Reaktionsansatzes mit verdünnte Salzsäure versetzt und mit Ethylacetat extrahiert; Laufmittel: Ethylacetat; die vollständige Umsetzung ist weiters am Farbumschlag der Reaktionslösung von dunkelrot auf braun erkennbar).
- Aussehen:: gelbe Kristalle
- Schmelzpunkt:: 340 °C
- Ausbeute:: 84%
- Summenformel:: C₁₁H₆ClN₅O₃ (291,66)
- IR (KBr):: 3274 (NH₂); 1687 (C=O)
- ¹H-NMR (DMSO-d₆):: 13,96 (s, 1H, NH); 13,02 (s, 1H, NH); 8,11-7,93 (m, 3H, Phenyl-H); 7,28 (s, 1H, Pyrazol-CH)

Die weitere Umsetzung zur Verbindung der allgemeinen Formel (1) erfolgt unter Bildung der Verbindungen der allgemeinen Formeln (E), (F) und (G) als Zwischenprodukte, welche ebenso wie die bereits beschriebenen Verbindungen (E-1), (F-1) und (G-1) hergestellt werden können.

### II. Verfahren zur Herstellung von Verbindungen des Typs (1) unter Variation des Substituenten R:

### II.1 Derivate mit unterschiedlichen Substituenten in Position 1

Vorstehend beschriebene und in Analogie zum Stand der Technik durchführbare Synthesestrategie ist zur Herstelllung der erfindungsgemäßen Verbindungen mit unterschiedlichen Substituenten in Position 1 anwendbar (für R ≠ H). Je nach Substitutionsmuster können weiterführende Derivatisierungen, wie dem Fachmann bekannte Verfahren der Oxidation, Reduktion; Etherspaltung, Acylierung, Alkylierung, Hydrolyse etc. vorgenommen werden. Die Herstellung der erfindungsgemäßen Verbindungen mit unterschiedlichen Substituenten in Position 1 wird anhand der nachfolgenden Beispiele erläutert, ist jedoch nicht auf diese Beispiele beschränkt. Vorgestellt werden hier Vertreter, welche einen Alkyl-, Cycloalkyl-, **Phenyl-, Pyridyl-, Thienyl oder Furyl**-Substituenten tragen, wobei dieser Rest direkt oder über einen Spacer mit dem Tricyclus verbunden ist.

### II. 1. 1 Beispiele für Derivate mit (substituiertem) Alkyl-Rest in Position 1

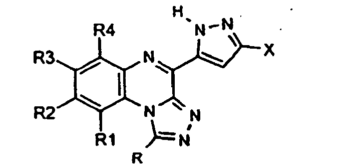

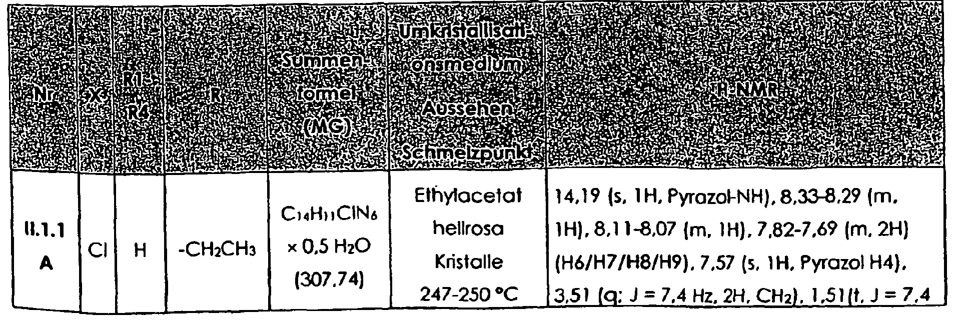

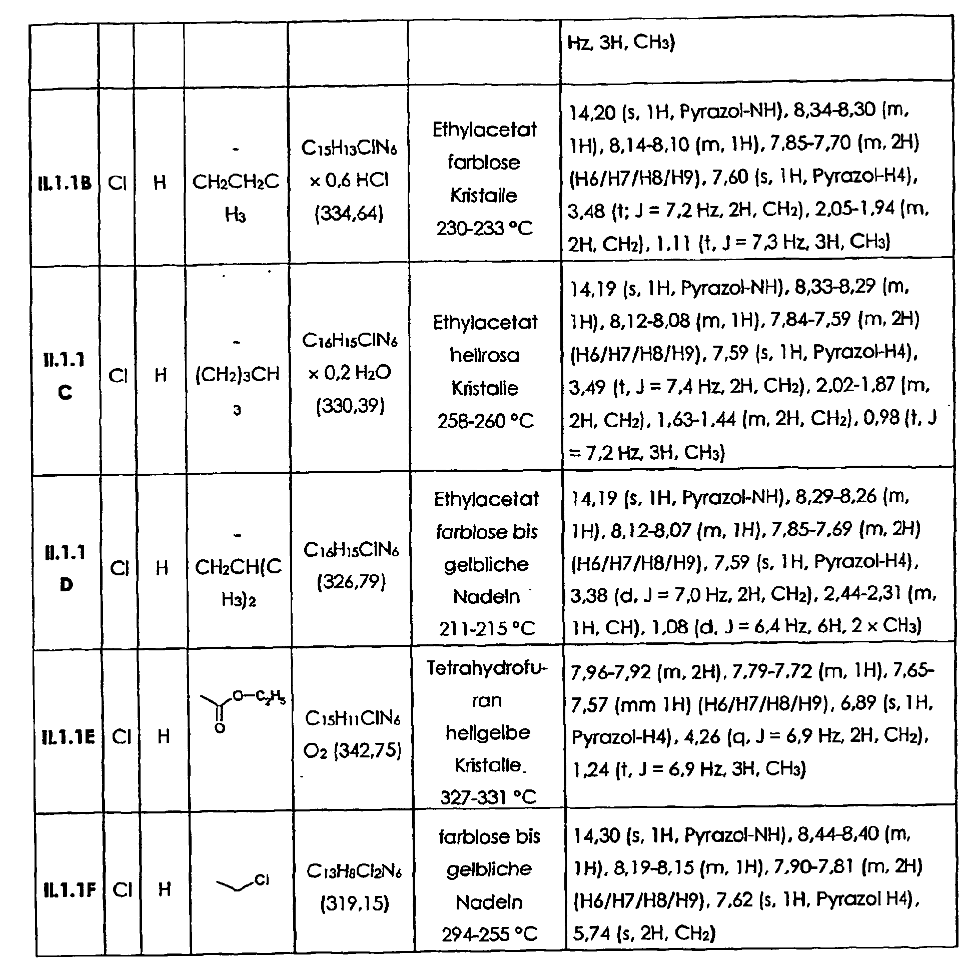

### II.1.2 Beispiele für Derivate mit Cycloalkyl-Substituenten in Position 1

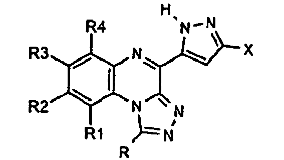

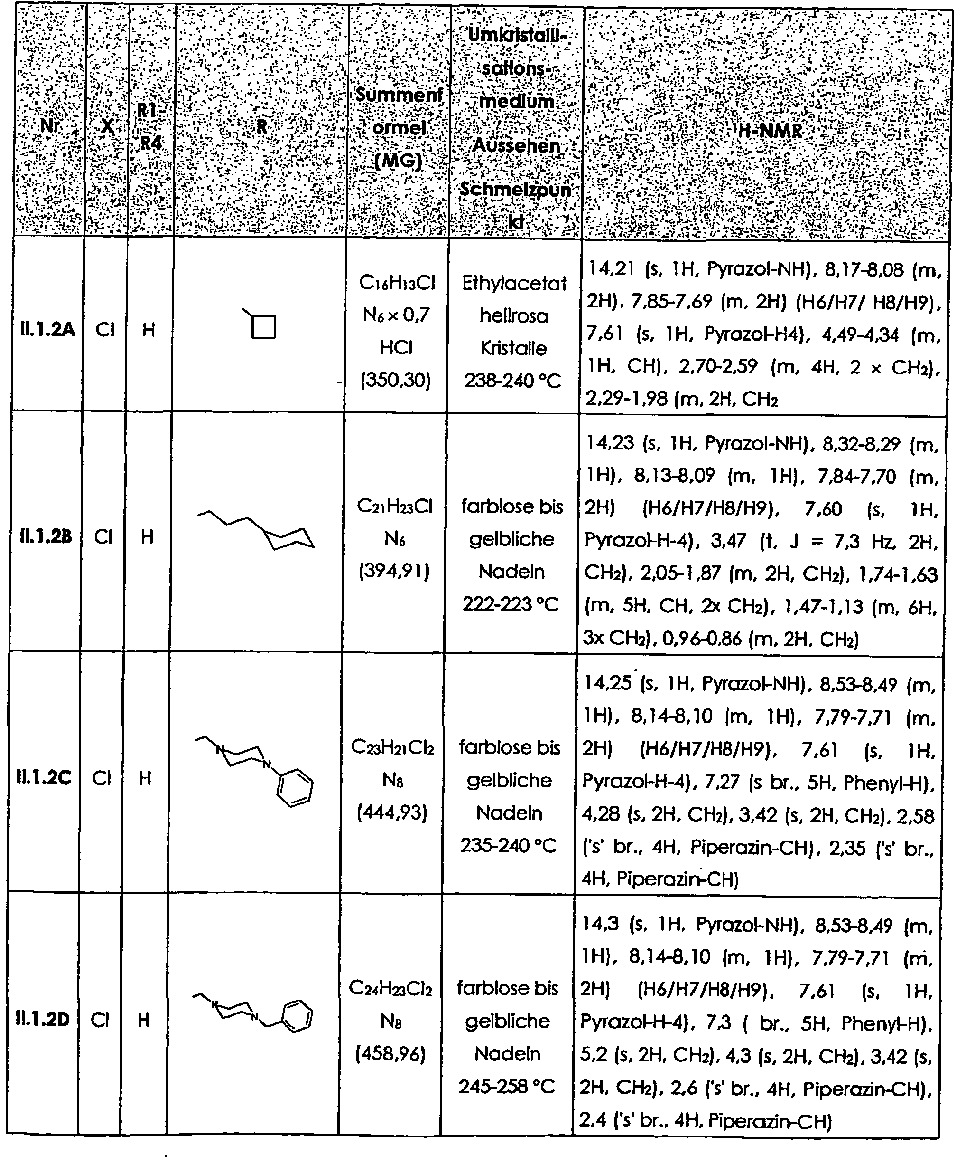

### II.1.3 A) Beispiele für Derivate mit (substituiertern) Phenyl-Rest in Position 1

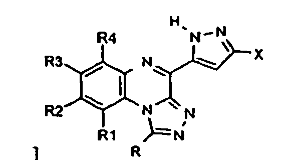

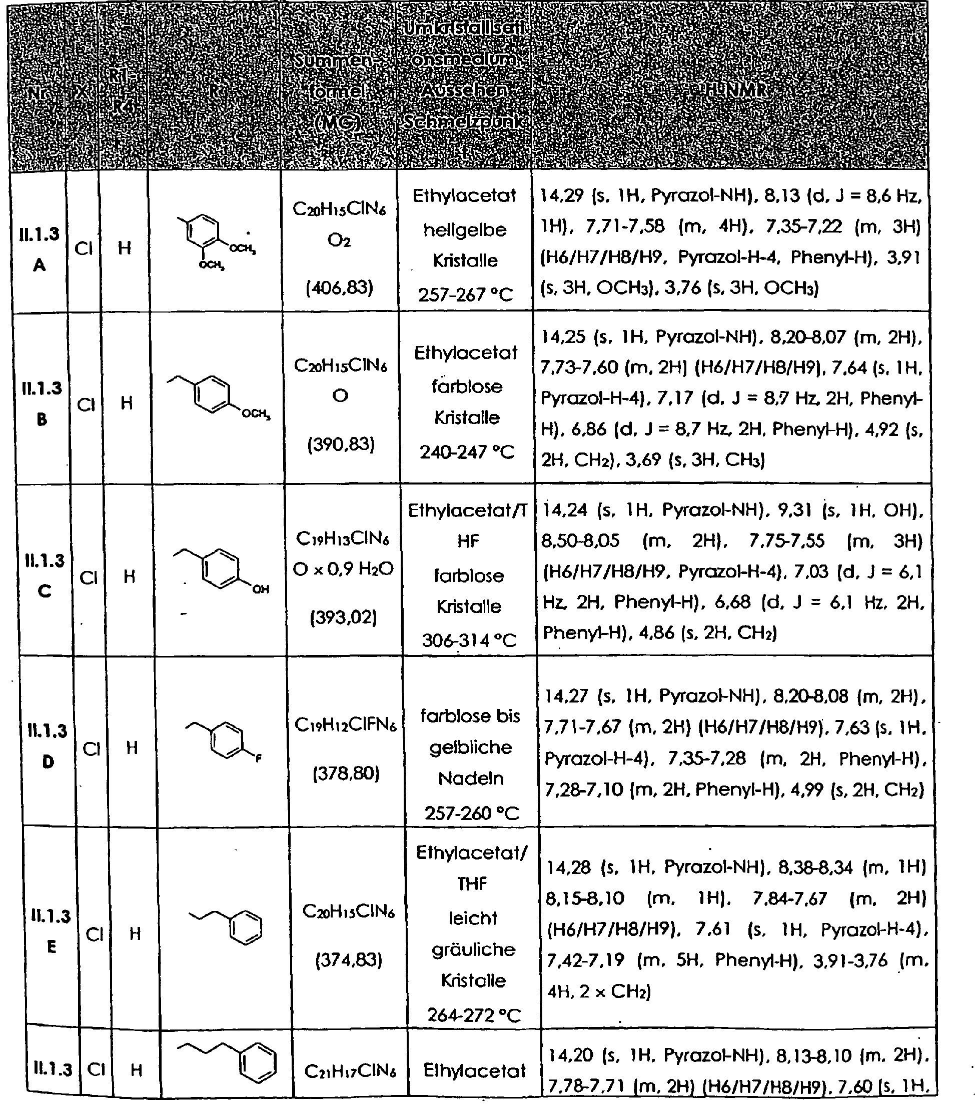

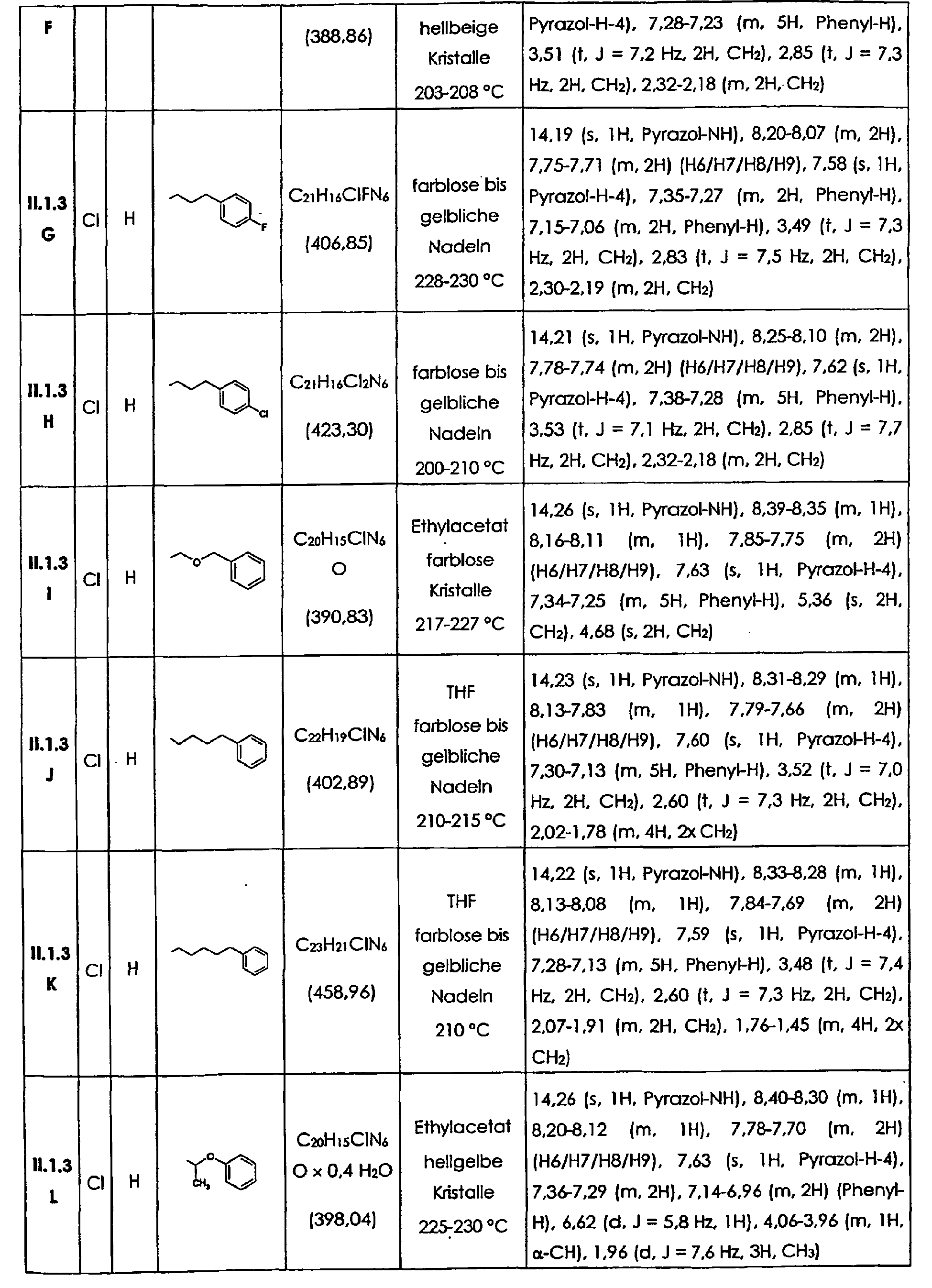

### II.1.3 B) Beispiele für Derivate mit Furyl - Thienyl- oder Pyridyl-Substituenten in Position 1

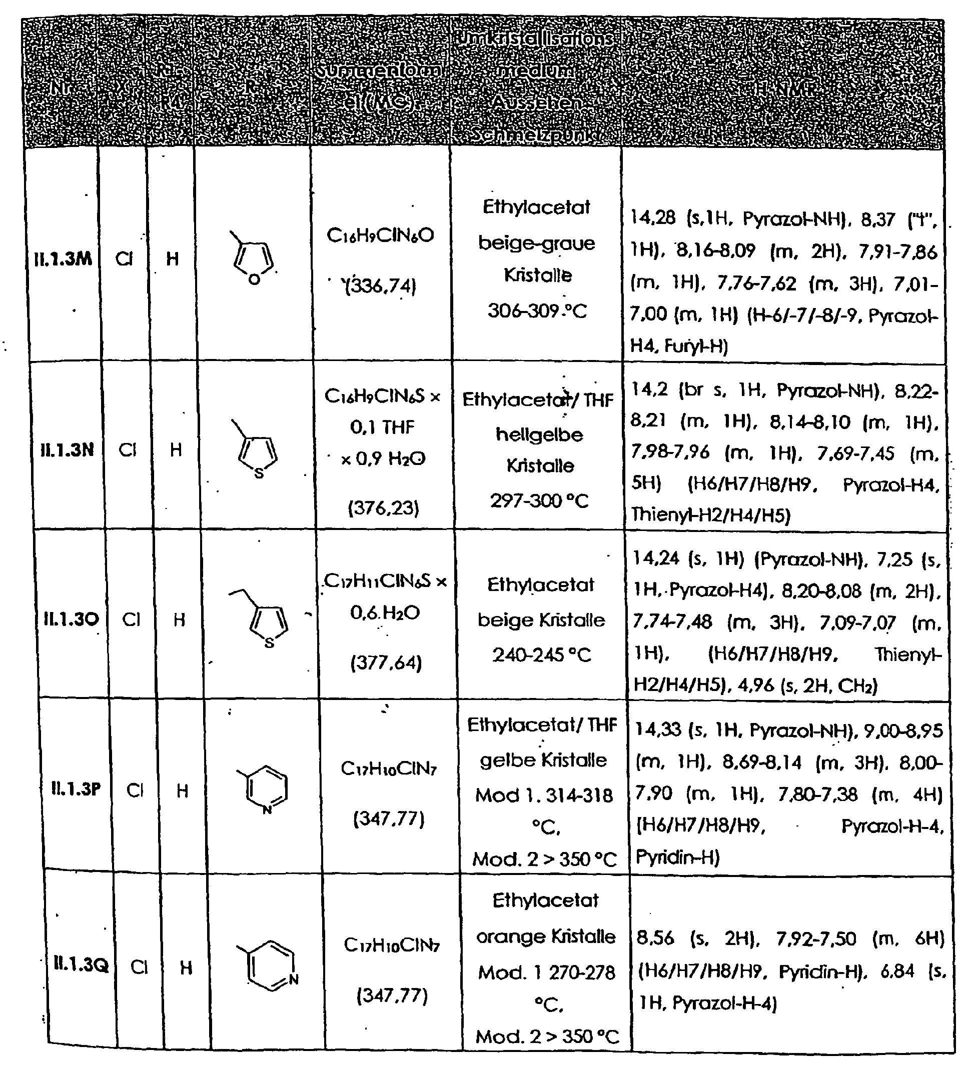

### II-2 Derivate der Verbindungen gemäß allgemeiner Formel (1) mit Wasserstoffatom in Position 1:

Derivate, bei denen der Substituent R (Position 1) ein Wasserstoffatom darstellt, können nicht nach der zuvor beschriebenen Methode erhalten werden. Diese werden beispielsweise durch Umsetzung der entsprechenden Hydrazino-Verbindung mit Orthoameisensäuretrimethylester umgesetzt. Im folgenden sind eine allgemeine Arbeitsvorschrift, welche die Bildung derartiger Derivate erlaubt, sowie drei Beispiele angeführt.

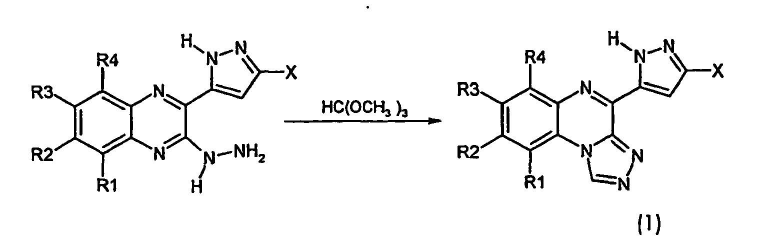

### Allgemeine Arbeitsvorschrift:

Eine Suspension aus 2,0 mmol der entsprechenden Hydrazinoverbindung in Orthoameisensäuretrimethylester (etwa 10 ml pro mmol) wird bis zur vollständigen Umsetzung unter Rückfluß erhitzt (DC-Kontrolle: einige Tropfen des Reaktionsansatzes werden mit Wasser versetzt, das Produkt wird mit Ethylacetat extrahiert, Laufmittel: Ethylacetat).

Nach dem Abkühlen auf Raumtemperatur wird das Reaktionsprodukt isoliert. Dies erfolgt entweder durch Abnutschen des auskristallisierten Produkts oder durch Versetzen des Reaktionsansatzes mit Wasser und Extraktion mit einem geeigneten organischen Lösungsmittel (beispielsweise Dichlormethan). Das isolierte Rohprodukt wird anschließend durch Umkristallisation aus einem geeigneten Lösungsmittel, beispielsweise Ethylacetat, Tetrahydrofuran, 1,4-Dioxan, *N,N-*Dimethylformamid oder Alkoholen gereinigt.

### II.2.A Synthese von 4-[3(5)Chlor-1H-pyrazol-5(3)-yl]-[1,2,4]triazolo[4,3-a]-chinoxalin (eine Verbindung der allgemeinen Formel (1) mit R = R1 = R2 = R3 = R4 = H, X = Cl)

- Reaktionszeit:: 2 Stunden
- Ausbeute:: 80 % (farblose Nadeln)
- Summenformel:: C₁₂H₇ClN₆ (270,68)
- Schmelztemperatur:: 302-304 °C (aus THF + 5 % DMF)
- ¹H-NMR (DMSO-d₆):: 14,24 (s, 1H, Pyrazol NH), 10,21 (s, 1H, H1), 8,49-8,44 (m, 1 H), 8,14-8,08 (m, 1 H), 7,88-7,70 (m, 2H) (H6, H7, H8, H9)

### II.2.B. Synthese von 4-13(5)-Chlor-1H-pyrazol-5(3)-yl-7,8-dimethyl-[1,2,4]triazolo[4,3-a]-chinoxalin (eine Verbindung der allgemeinen Formel (1) mit R = R1 = R4 = H, R2 = R3 = CH₃, X = Cl)

- Reaktionszeit:: 40 Minuten
- Ausbeute:: 90 % (farbloses Pulver)
- Summenformel:: C₁₄H₁₁ClN₆ (298,74)
- Schmeiztemperatur:: 320 - 323 °C (aus THF)
- ¹H-NMR (DMSO-d₆):: 14,13 (s, 1H, Pyrazol NH), 10,04 (s, 1H, H1), 8,17 (s, 1 H), 7,75 (s, 1H) (H6, H9), 7,56 (s, 1H. Pyrazol-H4), 2.41 (s, 3H, CH₃). 2,38 (s, 3H, CH₃)

### II.2.C. Synthese von 4-[3(5)-Chlor-1H-pyrazol-5(3)-yl]-7-methoxy-[1,2,4]triazolo[4,3-a]-chinoxalin (eine Verbindung der allgemeinen Formel (1) mit R = R1, R2, R4 = H, R3 = OCH₃, X = CI)

- Reaktionszeit:: 4 Stunden
- Ausbeute:: 67 % (gelbliches Pulver)
- Summenformel:: C₁₃H₉CIN₆O (300,71)
- Schmelztemperatur:: 287 - 290 °C (ab 200 °C Umwandlung in eine andere Modifikation) (aus Ethylacetat/THF)
- ¹H-NMR (DMSO-d₆):: 14,21 (s, 1 H, Pyrazol NH), 10,14 (s, 1H, H1), 8,40 (d, J = 9,0 Hz, 1H, H9), 7,64 (s, 1H, Pyrazol-H4). 7,55 (d, J = 2,9 Hz, 1H, H6), 7,47 (dd, J = 9,0 Hz, J = 2,9 Hz, 1 H, H8), 3,94 (s, 3H, OCH₃)

### II.3 Tetrazolochinoxaline der allgemeinen Formel (2), (1-Aza-Derivate):

Die Erfindung betrifft weiters neue Tetrazolochinoxaline, welche als *N*-Isostere der zuvor angeführten Triazolochinoxaline gemäß allgemeiner Formel (1) anzusehen sind, d.h. es wurde ein Ringkohlenstoff durch Stickstoff ersetzt. Derartige Derivate sind, wie anhand eines Beispiels aufgeführt, ausgehend von den entsprechenden Chlorchinoxalin-Abkömmlingen durch Umsetzung mit Natriumazid zugänglich.

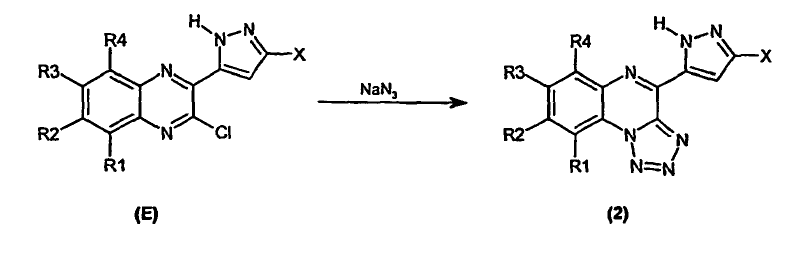

### Allgemeine Arbeitsvorschrift:

Äquimolare Mengen an entsprechendem Chlorchinoxalin-Derivat und Natriumazid werden in absolutem *N,N*-Dimethylformamid (ca. 10 ml pro mmol Chlorverbindung) bei 130°C bis zur vollständigen Umsetzung gerührt. Zur Aufarbeitung wird der Reaktionsansatz in verdünnte Salzsäure (etwa 100 ml pro mmol) gegeben, das kristallin anfallende Reaktionsprodukt wird isoliert, mit Wasser sowie Petrolether gewaschen und bis zur Gewichtskonstanz getrocknet.

Analysenreines Produkt wird durch Umkristallisation aus einem geeigneten Lösungsmittel erhalten.

### II.3.A. Synthese von 4-[3(5)-Chlor-1H-pyrazol-5(3)-yl]tetrazolo[1,5-a]chinoxalin (eine Verbindung der allgemeinen Formel (2) mit R1 = R2 = R3 = R4 = H, X = Cl)

Reaktionszeit: 5 Stunden
Ausbeute: 86 % (hellgelbe Kristalle)
Summenformel: C₁₁H₆ClN₇ (271,67)
Schmelztemperatur. 257-258 °C (aus Ethylacetat)
Elementaranalyse:

| | | C | H | N |
|---|---|---|---|---|
| | ber. | 48,63% | 2,23 % | 36,09% |
| | gef. | 48,45% | 2,17% | 35,98 % |

¹H-NMR (CDCl₃): 14,49 (s, 1 H, NH), 8,65-8,60 (m, 1 H), 8,31 - 8,27 (m, 1 H), 8,06 - 7,91 (m, 2H) (H6, H7, H8, H9), 7,62 (s, 1 H, Pyrazol H4)

Die in den nachfolgenden Abschnitten 111 und IV beschriebenen Strukturmodifikationen können ebenso auf das erfindungsgemäße N-Isoster der allgemeinen Formel (2) angewendet werden.

### III. Verfahren zur Herstellung von Verbindungen des Typs (1) bzw. (2) unter Variation des Substituenten R5:

Die Erfindung betrifft weiters neue pyrazolyl-substituierte [1,2,4]Triazolo[4,3-*a*]chinoxaline, bzw. 1-Aza-Isostere, welche dadurch gekennzeichnet sind, daß im Pyrazolring keine freie NH-Funktion mehr vorliegt, sondern einer der beiden Stickstoffatome einen weiteren Substituenten, vorzugsweise Alkyl, Allyl, (Hetero)Aralkyl oder Acyl trägt. Die Enführung dieses Substituenten R5 erfolgt bevorzugt durch nachträgliche Derivatisierung, beispielsweise Alkylierung, einer Verbindung der allgemeinen Formel (1), in welcher R5 gleich Wasserstoff ist:

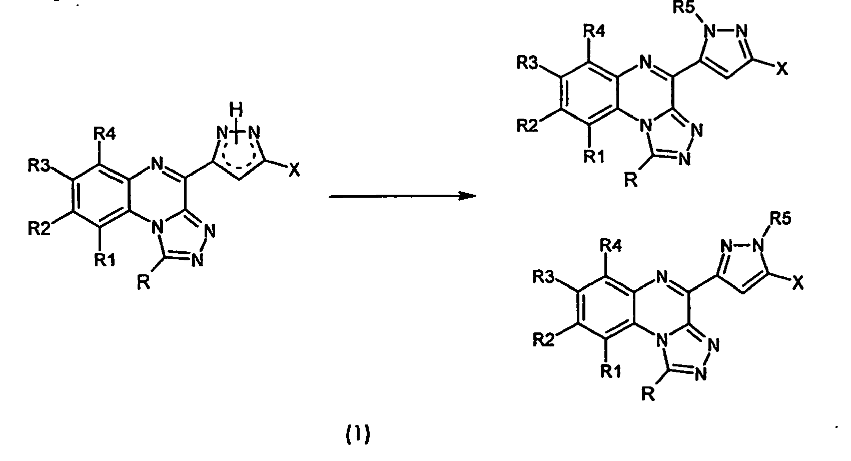

Bedingt durch die chemische Natur des Pyrazols ist die Bildung von R5-Stellungs-Isomeren zu erwarten, wobei durch Wahl der geeigneten Reaktionsbedingungen eines der R5-StellungsIsomere bevorzugt entstehen sollte. Gegebenenfalls auftretende Produktgemische lassen sich durch dem Fachmann bekannte Reinigungsverfahren, wie fraktionierte Kristallisation oder chromatographische Trennverfahren in die Isomeren-reinen R5-Substitutionsprodukte der allgemeinen Formel (1), wie oben dargestellt, auftrennen.

Anhand einiger Beispiele wird diese Strukturvariation dargestellt, wobei die Erfindung jedoch nicht auf dieses Beispiel beschränkt sein soll; ebenso können auch andere für Alkylierungsreaktionen beschriebene Arbeitsmethoden verwendet werden.

### Allgemeine Arbeitsvorschrift zur Alkylierung von N-unsubstituierten Derivaten der allgemeinen Formel (1)

Zu einer Lösung von einem Äquivalent des entsprechenden *N*-unsubstituierten Derivates der allgemeinen Formel (1) in etwa 5 ml/mmol eines absoluten Lösungsmittels (beispielsweise Dimethylsulfoxid, Tetrahydrofuran, 1,4-Dioxan, DMF) werden unter Stickstoffatmosphäre 2 Äquivalente Base (z.B. gepulvertes Kaliumhydroxid, Natrium, Natriumhydrid, Kalium-*tert*.-Butanolat) gegeben, wonach das Gemisch eine Stunde bei Raumtemperatur gerührt wird. Anschließend werden 2 Äquivalente des Alkylierungsmittels (bevorzugt Alkylhalogenid bzw. Mesylat) hinzugefügt, wonach das Reaktionsgemisch bis zur vollständigen Umsetzung bei Raumtemperatur gerührt wird (DC-Kontrolle: einige Tropfen des Reaktionsansatzes werden mit verdünnter Salzsäure versetzt und mit Ethylacetat extrahiert, Laufmittel: beispielsweise Dichlormethan/Ethylacetat = 19/1). Zur Aufarbeitung wird der Reaktionsansatz in verdünnte Salzsäure (etwa 100 ml/mmol) gegeben. Der entstandene Niederschlag wird abgenutscht und mit Wasser sowie Petrolether gewaschen oder durch Extraktion mit einem organischen Lösungsmittel extrahiert. Das getrocknete Rohprodukt wird anschließend durch Behandlung mit Aktivkohle vorgereinigt. Eine anschließende Reinigung / Auftrennung der Stellungs-Isomeren erfolgt chromatographisch, gegebenenfalls auch durch fraktionierte Kristallisation.

Eine eindeutige Strukturabsicherung ist bei diesen Derivaten beispielsweise mittels Röntgenstrukturanalyse möglich.

Unter Anwendung dieser allgemeinen Arbeitsvorschrift werden folgende Synthesebeispiele zur Herstellung von Verbindungen der allgemeinen Formel (1) durchgeführt:

### Synthese von 4-(5-Chlor-1H-1-methyl-pyrazol-3-yl)-1-(2-thienylmethyl)-[1,2,4]triazolo[4,3-a]-chinoxalin

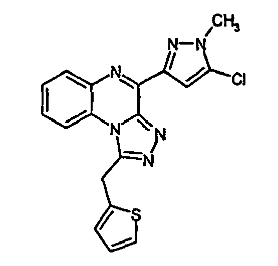

### Lösungsmittel: absolutes Dimethylsulfoxid

- Base:: gepulvertes Kaliumhydroxid
- Reaktionszeit:: 1.5 Stunden
- Aussehen:: gelbe, tafelig-rautenförmige Kristalle
- Summenformel:: C₁₈H₁₃CIN₆S (380,86)
- Schmelztemperatur:: 205-207 °C (aus Ethylacetat)
- ¹H-NMR (CDCl₃):: 8,32 - 8,27 (m, 1 H), 8,06 - 8,01 (m, 1 H), 7,66 - 7,50 (m, 2H) (H6, H7, H8, H9), 7,87 (s, 1 H, Pyrazol H4), 7,24 - 7,22 (m, 1 H), 6,95 - 6,90 (m, 1H), 6,81- 6,80 (m, 1 H) (Thiophen H3, H4, H5), 5,11 (s, 2H, CH₂), 4,08 (s, 3H, CH₃)

### Synthese von 4-[3(5)-Chlor-1-methyl-1H-pyrazol-5(3)-yl]-1-(3)-phenylpropyl)-[1,2,4]triazolo[4,3-a]-chinoxalin sowie Auftrennung in dessen Stellungsisomere

### Lösungsmittel: absolutes THF

- Base:: gepulvertes Kaliumhydroxid
- lsomerentrennung:: Säulenchromatographie:
stationäre Phase: Kieselgel
mobile Phase: 1,4-Dioxan + Diisopropylether = 1 + 1)

### 4-[5-Chlor-1-methyl-pyrazol-3-yl]-Isomer

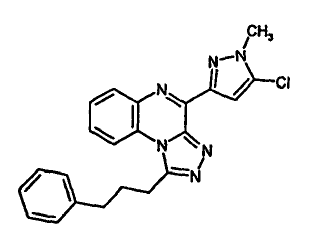

- Aussehen:: farblose Kristalle
- Reinigung:: Umkristallisation aus EA
- Summenformel:: C₂₂H₁₉ClN₆ (402,86)
- Ausbeute:: 0,145 g (32 %)
- Schmelzpunkt:: 179-180 °C
- IR [cm⁻¹]:: 3432, 3158, 2953 (NH)
- ¹H-NMR (CDCl₃):: 8,29 (d, J = 7,6 Hz 1H), 7,86 - 7,80 (m, 1H), 7,65 - 7,48 (m, 2H)) (H6, H7, H8. H9), 7,86 (s, 1 H, Pyraxol-H), 7,38 - 7,20 (m, 5H, Phenyl-H), 4,07 (s, 3H, CH₃), 3,50 (t, J = 7,5 Hz, 2H, CH₂), 2,93 (t, J = 7,0 Hz, 2H, CH₂), 2,48 - 2,33 (m, 2H, CH₂)

### 4-[3-Chlor-1-methyl-pyrazol-5-yl]-Isomer

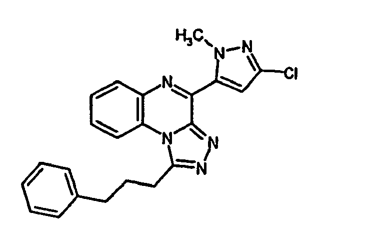

- Aussehen:: farblose Kristalle
- Reinigung:: Umkristallisation aus EA
- Summenformel:: C₂₂H₁₉ClN₆ (402,86)
- Ausbeute:: 0,260 g (58 %)
- Schmeizpunkt:: 158-160 °C
- IR [cm⁻¹]:: 3432 (NH)
- ¹H-NMR (CDCl₃):: 8,12 (dd, J = 8,1 Hz, J = 1,5 Hz, 1 H), 7,83 (dd, J = 8,1 Hz, J = 1,5 Hz, 1 H), 7,69 - 7,52 (m, 2H) (H6, H7, H8, H9), 7,88 (s, 1 H, Pyrazol-H), 7,38-7,21 (m, 5H, Phenyl-H), 4,40 (s, 3H, CH₃), 3,49 (t, J = 7,6 Hz, 2H, CH₂), 2,93 (t, J = 7,2 Hz, 2H, CH₂)

Eine Derivatisierung am Stickstoffatom des Pyrazolringes kann nicht nur auf der Stufe des Tri- oder Tetrazolochinoxalin-Derivates, sondern auch in einer früheren Reaktionsstufe, erfolgen. Im Fälleder gegenständlichen Verbindungen kann dieses ausgehend von Verbindungen des Typs **(D)**, **(E), (F)** oder **(G)** vorgenommen werden. Das nachfolgende Syntheseschema faßt die verschiedenen Möglichkeiten zusammen. Ein Verfahren zur Einführung einer Acylgruppe ist in der Literatur für (E) mit R1 bis R4 gleich Wasserstoff beschrieben (B. Matuszczak *et al., J. Heterocyclic Chem.* **35**:113-115, 1998). Ausgehend von Verbindungen des Typs **(D)** ist es bevorzugt, die Derivatisierung an einer Verbindung aufzunehmen, welche am Stickstoff eine Lactamschutz gruppe aufweisen.

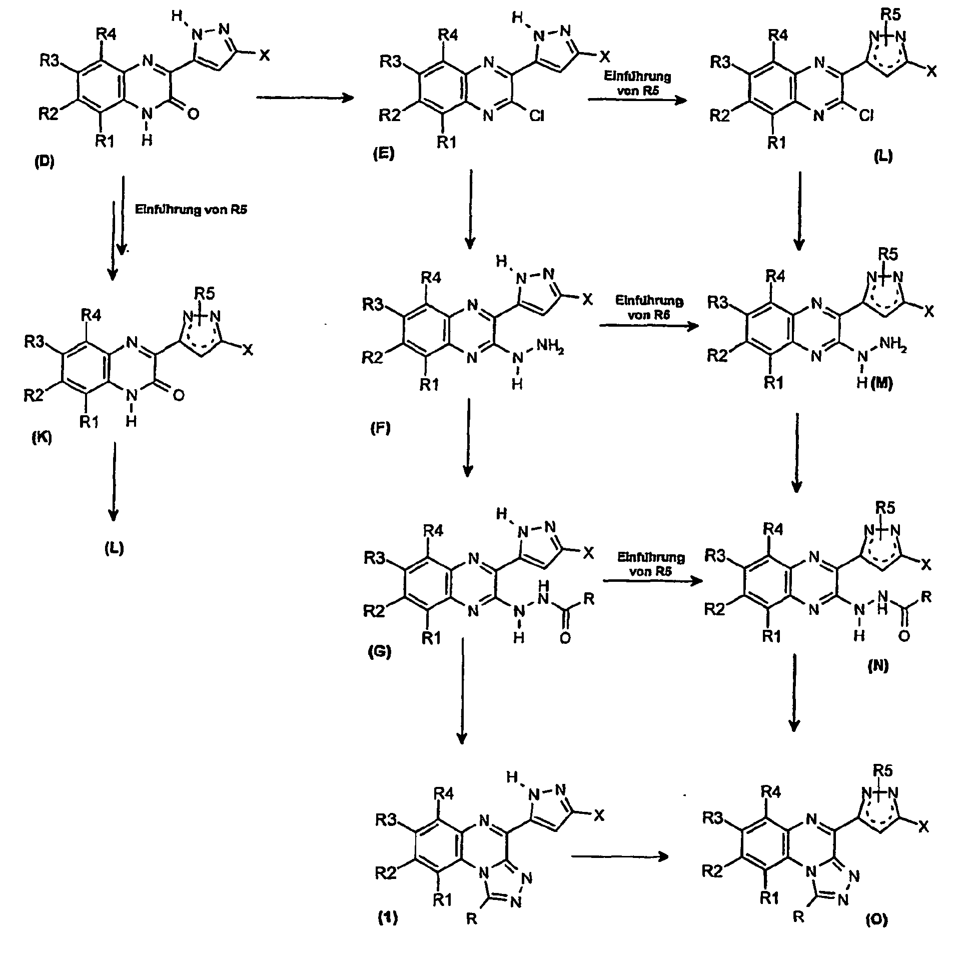

Die von **E** ausgehende Enführung von Substituenten R5 soll im folgenden anhand eines Beispiels näher beschrieben werden. Die gegenständliche Erfindung ist jedoch nicht darauf beschränkt, sondern kann, gegebenenfalls durch geringfügige Abänderung der Reaktionsbedingungen, auch problemlos auf andere Substituenten übertragen werden. Durch Variation der Bedingungen, insbesondere von Lösungsmittel, Base, Alkylierungsmittel, Temperatur und gegebenenfalls Katalysator kann das Verhältnis der beiden *N*-Isomere variiert und damit im Einzelfall auch zugunsten des gewünschten lsomers verschoben werden.

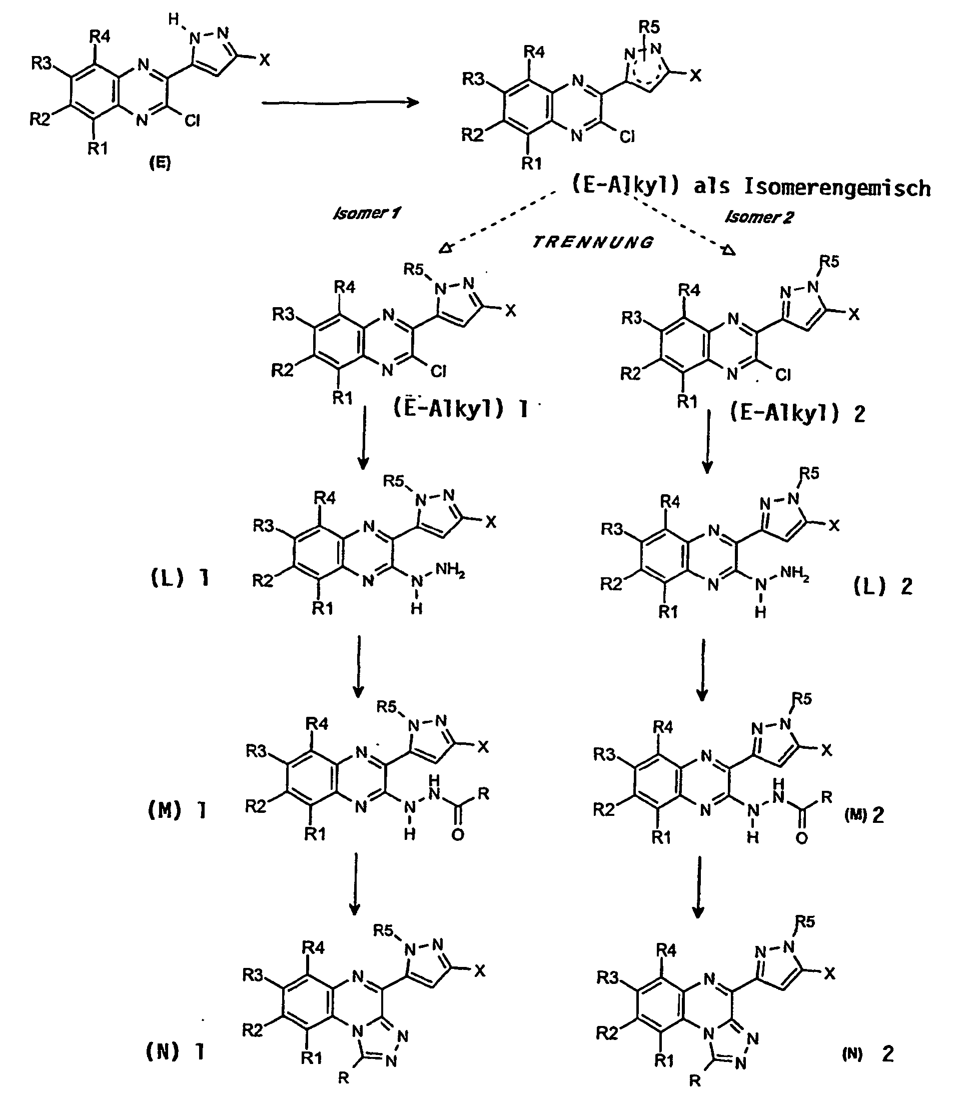

### Alkylierung eines 2-Chlor-3-[3(5)-chlor- 1H-pyrazol-5(3)-yl]-chinoxalin zur Herstellung von Verbindungen des Typs (E-Alkyl):

Zu einer Lösung bzw. Suspension von einem Äquivalent eines Chlorchinoxalins des Typs (E) in einem inerten Lösungsmittel (beispielsweise THF, DMF, 1,4-Dioxan, DMSO; jeweils bevorzugt absolutes Lösungsmittel) werden unter Stickstoffatmosphäre 2 Äquivalente einer Base (beispielsweise gepulvertes Kaliumhydroxid, Natrium, Natriumhydrid, Kalium-*tert*.-Butanolat) und bei Bedarf katalytische Mengen eines anorganischen lodids gegeben. Das Gemisch wird 1 Stunde bei Raumtemperatur gerührt. Bei Vorliegen einer Suspension kann sich eine Behandlung im Ultraschall günstig auf die Deprotonierung und damit auf den weiteren Reaktionsverlauf auswirken. Anschließend werden 2 Äquivalente des entsprechenden Alkylierungsmittels wie Alkylhalogenide oder Mesylate hinzugefügt, wonach das Reaktionsgemisch bis zur vollständigen Umsetzung gerührt wird (erforderliche Reaktionstemperatur: Raumtemperatur bis Siedebereich des eingesetzen Lösungsmittels; der Reaktionsverlauf wird mittels DC verfolgt, eine Probe des Reaktionsansatzes wird hierfür mit verdünnter Salzsäure versetzt und mit Ethylacetat extrahiert, Laufmittel: geeignetes Lösungsmittel bzw. -gemisch). Sofem erforderlich, d.h. bei unvollständiger Reaktion bzw, zu langsamem Verlauf können nachträglich noch Base und/oder Alkylierungsmittel zugegeben werden. Zur Aufarbeitung wird das Reaktionsgemisch in verdünnte Salzsäure gegeben und das Reaktionsprodukt isoliert. Dies erfolgt durch Abtrennung des entstandenen Niederschlages bzw. durch Extraktion mit einem organischen Lösungsmittel, wonach anschließend mit Wasser gewaschen wird. Die Trennung der Isomeren sowie deren Reinigung erfolgt mittels Chromatographie und/oder Umkristallisation.

Die allgemeine Arbeitsvorschrift wird anhand folgender Beispiele näher erläutert:

### A) Synthese von 2-Chlor-3-[3(5)-chlor-1-ethyl-1H-pyrazol-5(3)-yl]-chinoxalin mit R5 = Ethyl

- Base:: gepulvertes Kaliumhydroxid
- Alkylierungsmittel:: Ethyliodid
- Lösungsmittel:: absolutes THF
- Isomerentrennung:: Säulenchromatographie:
stationäre Phase: Kieselgel
mobile Phase: Dichlormethan + Ether = 49 + 1)
- Summenformel:: C₁₃H₁₀Cl₂N₄ (293,16)

### 1. Isomer:

- Aussehen:: farblose Kristalle
- Schmelztemperatur:: 149 °C (aus Diisopropylether)
- ¹H-NMR (CDCl₃):: 8,15-7,84 (m, 4H, H6, H7, H8, H9), 6,78 (s, 1 H, Pyrazol H4), 4,32 (q, J = 7,2 Hz, 2H, CH₂), 1,49 (t, J = 7,2 Hz. 3H, CH₃)

### 2. Isomer:

- Aussehen:: gelbe, tofelig-rautenförmige Kristalle
- Schmelztemperatur.: 126 °C (aus Diisopropylether)
- ¹H-NMR (CDCl₃):: 8,23 - 8,18 (m, 1 H), 8,06 - 8,01 (m, 1 H), 7,82 - 7,76 (m, 2H) (H6, H7, H8, H9), 7,04 (s, 1H, Pyrazol H4), 4,38 (q, J = 7,2 Hz, 2H, CH₂), 1,54 (t, J = 7,2 Hz, 3H, CH₃)

### B) Synthese von 2-Chlor-3-[3(5)-chlor-1-(2-methoxyethyl)-1H-pyrazol-5(3)-yl]-chinoxalin mit R5 = Methoxyethyl

- Base:: gepulvertes Kaliumhydroxid
- Alkylierungsmittel:: Methansulfonsäure-(2-methoxyethyl)ester
- Katalysator:: Kaliumiodid
- Lösungsmittel:: absolutes 1,4-Dioxan
- Isomerentrennung:: Säulenchromatographie:
stationäre Phase: Kieselgel
mobile Phase: Dichlormethan + Diisopropylether = 1 + 1)
- Summenformel:: C₁₄H₁₂Cl₂N₄O (323,18)

### 1. Isomer:

- Aussehen:: farblose Kristalle
- Schmelztemperatur:: 89 - 90 °C
- ¹H-NMR (CDCl₃):: 8,16-8,02 (m, 2H), 7,91 -7,79 (m, 2H) (H6, H7, H8, H9), 6,75 (s, 1 H, Pyrazol H4), 4,53 (t, J = 5,5 Hz, 2H, CH₂), 3,65 (t, J = 5,5 Hz, 2H, CH₂), 3,12 (s, 3H, CH₃)

### 2. Isomer:

- Aussehen:: farblose Nadeln
- Schmelztemperatur:: 125 °C
- ¹H-NMR (CDCl₃):: 8,22 - 8,16 (m, 1 H), 8,06 - 8,01 (m, 1 H), 7,83 - 7,74 (m, 2H) (H6, H7, H8, H9), 7,04 (s, 1H, Pyrazol H4), 4,48 (t, J = 5,8 Hz, 2H, CH₂), 3,88 (t, J = 5,8 Hz, 2H, CH₂), 3,76 (s, 3H, CH₃)

### Herstellung von Verbindungen des Typs L:

Zur Umsetzung wird eine Suspension des entsprechenden isomerenreinen substituierten 2-Chlor chinoxalins (E-Alkyl) in Hydrazinhydrat (ca. 10 ml/mmol) bis zur vollständigen Umsetzung unter Rückfluß erhitzt, wobei gegebenenfalls einige ml eines hochsiedenden Lösungsmittels, wie beispielsweise 1,4-Dioxan zugegeben werden (Reaktionskontrolle: eine Probe des Reaktionsansatzes wird mit Ethylacetat extrahiert und mit DC kontrolliert, Laufmittel: geeignetes Lösungsmittel bzw. -gemisch). Zur Aufarbeitung wird das Reaktionsgemisch in Eiswasser gegeben, und das Reaktionsprodukt durch Abtrennung des entstandenen Niederschlages bzw. durch Extraktion mit einem organischen Lösungsmittel isoliert und mit Wasser gewaschen. Analysenreine Produkte werden durch Umkristallisation aus einem geeigneten Lösungsmittel erhalten. Sofem erforderlich, wird das Rohprodukt zuvor chromatographisch gereinigt.

Diese allgemeine Arbeitsvorschrift wird anhand folgender Synthesebeispiele näher erläutert:

### Synthese von 3-[3(5)-Chlor-1-ethyl-1H-pyrazol-5(3)-yl]-2-hydrazinochinoxalin

- Summenformel:: C₁₃H₁₃ClN₆ (288,74)

### 1. lsomer:

- Aussehen:: gelbliche Kristalle
- Ausbeute:: 83 %
- Schmelztemperatur:: 141 - 143 °C
- ¹H-NMR (CDCl₃):: 7,96 - 7,91 (m, 1 H), 7,83 - 7,79 (m, 1 H), 7,72 - 7,64 (m, 1H), 7,54 - 7,45 (m, 1 H) (H6, H7, H8, H9), 6,62 (s, 1 H, Pyrazol H4), 4,33 (q, J = 7,2 Hz, 2H, CH₂), 4,18 (s br, 2H, NH₂), 1,47 (t, J = 7,2 Hz, 3H, CH₃)

### 2. isomer:

- Aussehen:: gelbliche Kristalle
- Ausbeute:: 96 %
- Schmelztemperatur:: 202 - 204 °C
- ¹H-NMR (CDCl₃):: 9,29 (s, 1H, NH), 7,92-7,87 (m, 1 H), 7,75-7,70 (m, 1H), 7,61-7,53 (m, 1 H), 7,53 - 7,35 (m, 1 H) (H6, H7, H8, H9), 7,17 (s, 1 H, Pyrazol H4), 4,34-4,23 (m, 4H, NH₂, CH₂), 1,52 (t, J = 7,2 Hz, 3H, CH₃)

### Herstellung von Verbindungen des Typs M und N:

### 1) Herstellung von Derivaten mit Wasserstoffatom in Position 1 durch Umsetzung der Verbindungen des Typs L mit Orthoameisensäureester

Die Darstellung erfolgt durch Umsetzung der isomerenreinen Hydrazine des Typs L gemäß allgemeiner Arbeitsvorschrift (sieheAbschnitt II.2) Dazu werden folgende Synthesebeispiele angeführt:

### Synthese von 4-[3(5)-Chlor-1-ethyl-1H-pyrazol-5(3)-y1]-[1,2,4]triazolo[4,3-a]-chinoxalin

- Summenformel:: C₁₄H₁₁ClN₆ (298,74)

### 1. Isomer:

- Aussehen:: farblose Kristalle
- Schmelztemperatur:: 282 - 284 °C (aus THF)
- ¹H-NMR (CDCl₃):: 9,36 (s, 1 H, CH), 8,19 -8,14 (m, 1H), 8,01 - 7,97 (m, 2H), 7,82 - 7,69 (m, 2H) (H6, H7, H8, H9, Pyrazol H4), 4,32 (q, J = 7,2 Hz, 2H, CH₂), 1,49 (t, J = 7,2 Hz, 3H, CH₃)

### 2. Isomer:

- Aussehen:: farblose Kristalle
- Schmelztemperatur:: 191-192 °C (aus Ethylacetat)
- ¹H-NMR (CDCl₃):: 9,36 (s, 1H, CH), 8,37 - 8,31 (m, 1H), 7,99 - 7,93 (m, 1H), 7,75 - 7,65 (m, 2H) (H6, H7, H8, H9), 7,88 (s, 1 H, Pyrazol H4), 4,46 (q, J = 7,3 Hz, 2H, CH₂), 1,57 (t, J = 7,3 Hz, 3H, CH₃)

### 11) Umsetzung der Verbindungen des Typs L mit Säurechloriden

Ausgehend von Verbindungen des Typs **L** können substituierte [1,2,4]Triazolo[4,3-*a*]chinoxaline nach der bereits zuvor beschriebenen Methode, d.h. Acylierung und anschließende Cyclisierung hergestellt werden (s. Herstellung von Verbindungen des Typs G-1 sowie des Typs (1)).

Die Herstellung erfolgt durch Umsetzung der isomerenreinen Hydrazine des Typs L gemäß allgemeiner Arbeitsvorschrift (sieheAbschnitt 11.2) oder durch Entopfverfahren, d.h. das Hydrazid wird nicht isoliert, sondern gleich direkt in den Tricyclus überführt. Obzwar dieses Verfahren erst an dieser Stelle beschrieben wird, so eignet es sich ebenso zur Herstellung pyrazolylunsubstituierter Derivate. Dazu beispielsweise wie folgt:

### Synthese von 4-[3(5)-Chlor-1-ethyl-1H-pyrazol-5(3)-yl]-1-(3-phenylpropyl)-[1,2,4]triazolo[4,3-a]-chinoxalin

- Summenformel:: C₂₃H₂₁ClN₆ (416,92)

### 1. Isomer:

Herstellung im Eintopfverfahren unter Verwendung von Acetonitril als Lösungsmittel
- Aussehen:: farblose Kristalle
- Schmelztemperatur:: 175 - 176 °C (aus Ethylacetat)
- ¹H-NMR (CDCl₃):: 8,13-8,08 (m, 1H), 7,89 (s, 1H), 7,86-7,81 (m, 1H), 7,69-7,53 (m, 2H) (H6, H7, H8, H9, Pyrazol H4), 7,38 - 7,21 (m, 5H, Phenyl-H), 4,85 (q, J = 7,2 Hz, 2H, CH₂), 3,49 (t, J = 7,8 Hz, 2H, CH₂), 2,94 (t, J = 7,1 Hz, 2H, CH₂), 2,48-2,33 (m, 2H, CH₂), 1,55 (t, J = 7,2 Hz, 3H, CH₃)

### 2. Isomer:

Herstellung in 2 Stufen, nämlich Acylierung und Cyclisierung unter Verwendung von Acetonitril als Lösungsmittel
- Aussehen:: farblose Nadeln
- Schmelztemperatur:: 146 - 147 °C (aus Diisopropylether)
- ¹H-NMR (CDCl₃):: 8,33 - 8,29 (m, 1 H), 7,86 - 7,80 (m, 2H), 7,66 - 7,48 (m, 2H) (H6, H7, H8, H9, Pyrazol H4), 7,38 - 7,20 (m, 5H, Phenyl-H), 4,44 (q, J = 7,3 Hz, 2H, CH₂), 3,50 (t, J = 7,7 Hz, 2H, CH₂), 2,94 (t, J = 7,1 Hz, 2H, CH₂), 2,48-2,34 (m, 2H, CH₂), 1,55 (t, J = 7,3 Hz, 3H, CH₃)

### IV. Verfahren zur Herstellung von Verbindungen des Typs (1) bzw. (2) unter Variation des Restes X:

Die Erfindung betrifft weiters pyrazolyl-substituierte [1,2,4]Triazolo[4,3-*a*]chinoxaline oder Tetrazoloderivate, in welchen das am Pyrazolring gebundene Chloratom durch andere Halogene bzw. durch Wasserstoff ersetzt ist. Die Enthalogenierung kann beispielsweise reduktiv erfolgen, d.h. durch Umsetzung in Wasserstoffatmosphäre oder mit einem Wasserstoff-Lieferanten wie Ammoniumformiat in Gegenwart eines geeigneten Katalysators (beispielsweise Palladium auf Aktivkohle) (B. Matuszczak, K. Mereiter, '*Syntheses in the Series of Pyrazolyl-substituted Quinoxalines', Heterocycles,* **45**, 2449-2462 (1997)). Begünstigt wird diese Enthalogenierungsreaktion durch Umsetzung bei erhöhter Temperatur: Als Lösungsmittel eignen sich vor allem Alkohole, Tetrahydrofuran sowie 1,4-Dioxan.

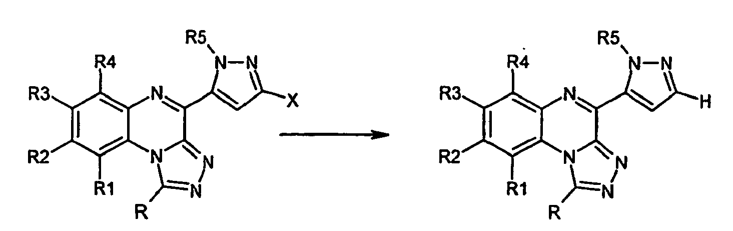

Gegebenenfalls können die Verbindungen in ihre Salze, insbesondere für die pharmazeutische Anwendung, in ihre physiologisch verträglichen Salze mit einer anorganischen oder organischen Säure, übergeführt werden. Als Säuren kommen hierfür beispielsweise Bemsteinsäure, Bromwasserstoff, Essigsäure, Fumarsäure, Maleinsäure, Methansulfonsäure, Milchsäure, Phosphorsäure, Salzsäure, Schwefelsäure, Weinsäure oder Citronensäure in Betracht. Ferner können Mischungen der vorgenannten Säuren eingesetzt werden.
Sofern im Molekül eine acide Funktion vorliegt, kann diese Verbindung für die pharmazeutische Anwendung auch in ein physiologisch verträgliches Salz übergeführt werden. Die Gegenionen können sowohl anorganischer als auch organischer Natur sein. Es handelt sich hierbei um Ionen aus Alkalimetallen, Erdalkalimetallen, Ammonium sowie Ammonium-Abkömmlingen, in welchen anstelle von Wasserstoff ein bis vier organische Substituenten, vorzugsweise Alkylreste vorliegen.

Des weiteren können die Verbindungen auch in Form von physiologisch verträglichen Solvaten, insbesondere von Hydraten vorliegen.

im folgenden Abschnitt sind einige Resultate der biologischen Testung von ausgewählten Vertretem aufgeführt. Die Verbindungsnummer entspricht der im synthetischen Teil beschriebenen Nummer.

### Biologische Tests der erfindungsgemäßen Verbindungen gemäß allgemeiner Formel (1) oder (2) sowie deren Auswertung:

Die Daten der nachfolgend aufgeführten biologischen Tests resultieren aus Radioligand-Bindungs-Assays an A₁, A_{2A}, A_{2B} und A₃ Adenosinrezeptoren (ARs).

Folgende selektive Radioliganden werden bei den biologischen Tests eingesetzt:
- [³H]CCPA: (für A₁ ARs) [Klotz *et al., Naunyn-Schmiedeberg's Arch. Pharmacol., **340,*** 679 (1989)]
- [³H]MSX-2: (für A_{2A} ARs) [Müller *el al., Eur. J. Pharm. Sci ,* **10**, 259 (2000)]
- [³H]ZM241385: (für A_{2B} ARs) [X.-D. Ji, K.A. Jacobson, *Drug Des. Discov.,* **16**, 217 (1999)]
- [³H]PSB-11: (neuer antagonistischer Radioligand für humane A₃ ARs) [M. Dieckmann, M. Thorand, V. Ozola, C.E. Müller, in Vorbereitung (2001)]
- [¹²⁵|]AB-MECA: (für Ratten-A₃ ARs) [Olah *et al., Mol. Pharmacol.,* **45**, 978 (1994)].

### Beschreibung der verwendeten Assays zur Testung der Substanzen

### Herstellung von Gewebepräparationen aus Rattenhirn

Für die Präparationen werden tiefgefrorene Rattenhime der Fa. Pel Freez (Rogers, Arkansas, USA), verwendet. Die Rattenhime werden in 0,32 M Saccharose-Lösung aufgetaut, die Cortex wird abgeschnitten und die Striata werden herauspräpariert.

Der Zellaufschluß der Hirnrinde (Cortex) erfolgt mit einem Glas-Teflon-Homogenisator (Stufe 5-6, 10 sec) in 0,32 M Saccharose-Lösung. Die Membranen werden über verschiedene Zentrifugationsschritte aufgereinigt. Mit einer Zentrifugation bei 600 *g,* 4 °C, 10 Minuten werden über die Pl-Fraktion grobe Zelltrümmer abgetrennt und der Überstand (P2-Fraktion) wird weiter aufgearbeitet: Bei den nachfolgenden drei Zentrifugationsschritten (35.000 *g*, 4 °C; 60 Minuten) entsteht ein Pellet, das in 50 mM TRIS-Puffer pH 7,4 mit dem Ultra Turrax (Stufe 3-4, 3 s) resusperidiert wird; der jeweilige Überstand wird verworfen. Die Striata werden in kalten TRIS-Puffer gegeben, mit dem Ultra-Turrax auf Stufe 3 bis 4 homogenisiert (3 sec) und anschliessend bei 35.000 g, 4 °C, 20 Minuten zentrifugiert. Der Überstand wird verworfen und das Pellet in 50 mM TRIS-Puffer (50 mM, pH 7,4) resuspendiert. Der Waschvorgang wird noch zweimal wiederholt. Die Membranpräparationen werden bei -80 °C aufbewahrt und sind mehrere Monate lang haltbar. Die Himcortex-Membranpräparation wird für A₁-, das Striatum für A_{2A}-Radioligand-Bindungsstudien verwendet.

### Zellkultur:

Als Zellkulturen werden beispielsweise CHO-Zellen, die den humanen A₁-Adenosinrezeptor exprimieren, verwendet.

Das Nährmedium, Trypsin und PBS (Phosphat-gepufferte Kochsalzlösung) werden im Wasserbad auf 37 °C erwärmt. Das Medium wird aus der zu ungefähr 80 % bis 90 % mit CHO-Zellen konfluent bewachsenen Flasche abgesaugt. Man gibt PBS-Puffer zu und läßt diesen etwa 5 Minuten einwirken. Danach wird der Puffer abgesaugt und die Zellen werden mit Trypsin/EDTA-Lösung bedeckt. Nach etwa 5 Minuten lösen sich die Zellen vom Flaschenboden ab. Die Trypsin/EDTA-Lösung wird mit mindestens der gleichen Menge an Medium verdünnt. Mit diesem Gemisch wird der Flaschenboden zwei- bis dreimal abgespült, um noch anhaftende Zellen abzulösen.

Anschließend wird die Suspension in ein Zentrifugenglas gegeben und bei 20°C 5 Minuten lang bei 1.000 *g* zentrifugiert. Der Überstand wird abgesaugt und das Zellpellet in frischem Medium aufgenommen. Die Zellsuspension wird auf 20 bis 25 beschriftete und mit Medium befüllte Zellkulturflaschen gleichmäßig verteilt. Die Flaschen werden leicht geschüttett, um die Zellen gut zu verteilen, und anschließend im Brutschrank (37°C; 5 % CO₂) 2 bis 3 Tage lang inkubiert.

Es wird beispielsweise folgende Mediumzusammensetzung für CHO-Zellen verwendet:

DMEM F12 mit den folgenden Zusätzen:
● 10 % fötales Kälberserum
● 1 % Penicillin/Streptomycin (fertige Mischung mit 10.000 U Penicillin und 10 mg Streptomycin)
● 2 mM L-Glutamin (Stammlösung: 200 mM in 0,85 % NaCl-Lösung)
● 0,2 mg/mL G418 (Genticinsulfat)

Für CHO-Zellen, die den A_{2A}-Adenosinrezeptor exprimieren , werden die Arbeitsschritte analog zur vorgenannten A₁-Adenosinrezeptor-CHO-Kultur durchgeführt. Dem Medium wird 0,5 U/mL Adenosindesaminase zugesetzt.

Weiters werden A_{2B}-Adenosinrezeptor-Membranen bereitgestellt, wobei beispielsweise fertig präparierte Membranen von HEK-Zellen, die den humanen A_{2B}-Rezeptor in hoher Dichteexprimieren (ca. 4 pmol/mg Protein) käuflich erworben wurden (Receptor Biology USA via Perkin Elmer Life Sciences, Köln, Deutschland).

Für CHO-Zellen, die den A₃-Adenosinrezeptor exprimieren , werden ebenso die Arbeitsschritte analog zur A₁-Adenosinrezeptor-CHO-Kultur durchgeführt. Das Medium wird, wie oben beschrieben, eingesetzt.

### Membranpräparation von rekombinanten humanen Rezeptoren:

Bereitstellung rekombinanter humaner A₁-Adenosinrezeptoren exprimiert von CHO-Zellen für A₁₋Adenosinrezeptor-Bindungsstudien:

Auf Gewebekulturschalen werden 2 bis 3 ml eiskalter TRIS-Puffer (pH 7,4; 50 mM) gegeben. Mit einem Gummischaber werden die Zellen von den Platten abgeschabt und in einem Becherglas aufgefangen. Die Platten werden mit weiteren 1 bis 2 ml TRIS-Puffer (pH 7,4; 50 mM) nachgespült und in ein Becherglas gegeben. Die Zellsuspension wird auf Zentrifugenröhrchen verteilt, mit dem Homogenisator auf höchster Stufe homogenisiert (3 bis 4 sec) und bei 4°C, 35.000 *g* 20 Minuten zentrifugiert. Der Überstand wird verworfen und das Pellet in TRIS-Puffer (pH 7,4; 50 mM) mit dem Glas-Teflon-Homogenisator auf höchster Stufe 3 bis 4 sec resuspendiert. Diese Suspension wird erneut zentrifugiert (4 °C; 35.000 g, 20 Minuten). Der Waschvorgang wird noch ein weiteres Mal wiederholt. Das erhaltene Pellet wird mit möglichst wenig TRIS-Puffer (pH 7,4; 50 mM) resuspendiert und in Aliquots (0,25 mL und 0,5 mL) abgefüllt.

Bereitstellung rekombinanter humaner A_{2A}-Adenosinrezeptoren exprimiert in CHO-Zellen für A_{2A-}Adenosinrezeptor-Bindungsstudien:

Auf Gewebekulturschalen werden 2 bis 3 ml eiskalter TRIS-Puffer (pH 7,4; 50 mM) gegeben. Mit einem Gummischaber werden die Zellen von den Platten abgeschabt und in einem Becherglas aufgefangen. Die Platten werden mit weiteren 1 bis 2 ml TRIS-Puffer (pH 7,4; 50 mM) nachgespült. Die Zellsuspension wird auf Zentrifugenröhrchen verteilt und bei 4°C, 35.000 *g* 20 Minuten zentrifugiert. Der Überstand wird verworfen und das Pellet in TRIS-Puffer (pH 7,4; 50 mM) mit dem Homogenisator auf höchster Stufe 3 bis 4 sec resuspendiert. Diese Suspension wird erneut zentrifugiert. Der Waschvorgang wird noch ein weiteres Mal wiederholt. Das erhaltene Pellet wird mit möglichst wenig TRIS-Puffer (pH 7,4; 50 mM) resuspendiert und in Aliquots (0,25 mL und 0,5 mL) abgefüllt.

Bevor das Gewebe im Assay eingesetzt wird, muß es erneut mit TRIS-Puffer (pH 7,4; 50 mM) 20 Minuten bei 4 °C und 35.000 g gewaschen werden. Die unspezifische Bindung kann dadurch verringert werden.

### Bereitstellung rekombinanter humaner A₃-Adenosinrezeptoren exprimiert in CHO-Zellen für A₃₋Adenosinrezeptor-Bindungsstudier

Auf Gewebekulturschalen werden 2 bis 3 ml eiskalter TRIS-Puffer (pH 7,4; 50 mM) gegeben. Mit einem Gummischaber werden die Zellen von den Platten abgeschabt und in einem Becherglas aufgefangen. Die Platten werden mit weiteren 1 bis 2 ml TRIS-Puffer (pH 7,4; 50 mM) nachgespült. Die Zellsuspension wird auf Zentrifugenröhrchen verteilt und bei 4 °C, 35.000 *g* 20 Minuten zentrifugiert. Der Überstand wird verworfen und das Pellet in TRIS-Puffer (pH 7,4; 50 mM) mit dem Homogenisator auf höchster Stufe 3 bis 4 sec resuspendiert. Diese Suspension wird erneut zentrifugiert. Der Waschvorgang wird noch ein weiteres Mal wiederholt. Das erhaltene Pellet wird mit möglichst wenig TRIS-Puffer (pH 7,4; 50 mM) resuspendiert und in Aliquots (0,25 mL und 0,5 mL) abgefüllt.

### Radioligand-Rezeptor-Bindungsstudien:

Die Testsubstanzen werden in DMSO zu 10 mM gelöst. Die Endkonzentration an DMSO im Assay beträgt 2,5% für A₁- und A_{2A}- Adenosinrezeptor-Assays und 1-2% für A_{2B}- und A₃-Assays. Aus der Stammlösung werden Verdünnungsreihen der Testsubstanzen hergestellt. Man verwendet 5-8 verschiedene Testkonzentrationen, die 3 Grössenordnungen umfassen (z.B. 1 nM bis 1000 nM).

### Radioligand-Bindungsstudien mit [³H]CCPA an A₁-Adenosinrezeptoren:

In 1 mL Gesamtvolumen sind 25 µL DMSO (Gesamtbindung) oder 25 µL 2-Chloradenosin (400 µM in DMSO, unspezifische Bindung) oder 25 µL Testsubstanz in DMSO sowie 775 µL TRIS-Puffer, 50 mM, pH 7,4; 100 µL [³H]CCPA (Endkonzentration: 0,5 nM; K_{D}-Wert: 0,2 nM) und 100 µL Rattencortex-Homogenat (70 µg/mL) oder eine Membranpräparation aus CHO-Zellen, die rekombinant humane A₁-Adenosinrezeptoren exprimieren (30-50 g/mL) in 50 mM TRIS-Puffer, pH 7,4, versetzt mit 0,12 I.U. ADA, enthalten. Die Mischung wird 90 Minuten lang in einem Schüttelwasserbad bei 23 °C inkubiert. Anschliessend filtriert man mit einem Brandel Zellemtegerät ab und wäscht mit eisgekühltem TRIS-Puffer, 50 mM, pH 7,4 (3 mL) zweimal nach. Die Filterscheiben werden in Szintillations-Vials überführt, mit 2,5 mL Ultima Gold Szintillationscocktail übergossen und mind. 6 Stunden lang inkubiert bevor sie im Szintillations-Counter gemessen werden.

Rezeptorbindungsassay mit [³H]MSX-2 an A_{2A}-Adenosinrezeptoren :

| | |
|---|---|
| 25 µL | DMSO, NECA (800 µM) bzw. Testsubstanz-Verdünnungen |
| 775 µL | TRIS-Puffer (Herstellung s.o.) |
| 100 µL | [³H]MSX-2 (Endkonzentration 1 nM; K_{D}-Wert 8 nM) |
| 100 µL | Ratten-Striatum-Membranpräparation oder Präparation von CHO-Zellen, die den humanen A_{2A}-Rezeptor exprimieren, inkubiert mit 0,12 IE Adenosindesaminase (Proteinkonzentration: 25-75 µg/mL) |
| 1000 µL | Endvolumen |

Der Assay wird gemäß der Vorschrift des Rezeptor-Bindungsassays mit [³H]CCPA unter Berücksichtigung der oben aufgelisteten Mengen und Lösungen durchgeführt. Die Inkubationszeit beträgt 30 Minuten bei 23°C im Schüttelwasserbad. Der Glasfaserfilter wird vor Gebrauch 30 Minuten in 0,5 % PEI-Lösung eingelegt.

Rezeptorbindungsassay mit [³H]ZM241385 an rekombinanten A_{2B}-Adenosinrezeptoren exprimiert in HEK-Zellen:

| | |
|---|---|
| 10 µL | DMSO, NECA (1 mM) bzw. Testsubstanz-Verdünnungen |
| 790 µL | 10 mM HEPES-Puffer, pH 7,4 (Herstellung s.o.) |
| 100 µL | [³H]ZM241385 (Endkonzentration 5 nM; K_{D}-Wert 33 nM) |
| 100 µL | Membranpräparation, mindestens 15 Minuten inkubiert mit 0,12 IE Adenosindesaminase (Proteinkonzentration: 40 µg/mL) |
| 1000 µL | Endvolumen |

Die Membranen werden aufgetaut und gemäß der Vorschrift unter Rezeptor-Bindungsassays mit [³H]CCPA durchgeführt unter Berücksichtigung der oben aufgelisteten Mengen und Lösungen. Die Verdünnung im Assay beträgt 1:100 (um den DMSO Gehalt auf 1 % zu reduzieren, da sich eine höhere DMSO-Konzentration ungünstig auswirkt). Die Inkubationszeit beträgt 30 Minuten bei 23 °C im Schüttelwasserbad. Der Glasfilter wird vor Gebrauch 30 Minuten in 0,5 % PEI-Lösung eingelegt.

Rezeptorbindungsassay mit [³H]PSB-11 an rekombinanten A₃-Adenosinrezeptoren exprimiert in CHO-Zellen:

| | |
|---|---|
| 10 µL | DMSO, R-PIA (1 00 µM) bzw. Testsubstanz-Verdünnungen |
| 350 µL | 50 mM TRIS-Puffer, pH 7,4 (Herstellung s.o.) |
| 40 µL | [³H]PSB-11 (Endkonzentration 0,5 nM; K_{D}-Wert 2 nM) |
| 100 µL | Membranpröparation (mindestens 15 Minuten) inkubiert mit 0,12 IE Adenosindesaminase (Proteinkonzentration: 100 µg/mL) |
| 500 µL | Endvolumen |

Der Assay wird gemäß der Vorschrift unter Rezeptor-Bindungsassays mit [³H]CCPA unter Berücksichtigung der oben aufgelisteten Mengen und Lösungen durchgeführt. Die Verdünnung im Assay beträgt 1:50. Der DMSO Gehalt ist hier 2 %. Die Inkubation bei 23 °C im Schüttelwasserbad dauert 45 Minuten.

### [³⁵S]GTPγS-Assay:

| | |
|---|---|
| 10 µL | DMSO, GTPγS bzw. Testsubstanz-Verdünnungen |
| 150 µL | TRIS-Puffer 50 mM pH 7,4 für [³⁵S]GTPγS-Assay (s.o.) |
| 20 µL | [³⁵S]GTPγS (Endkonzentration 0,1 bis 0,5 nM; entspricht - 1250 Ci/mmol) |
| 20 µL | Membranpräparation inkubiert mit 0,12 IE Adenosindesaminase (Proteinkonzentration: 75 µg/mL) |
| 200 µL | Endvolumen |

Zunächst werden Lösungen der Testsubstanzen in DMSO in den benötigten Konzentrationen hergestellt. Die Kurve besteht aus 7 bis 8 Messpunkten (gemessen jeweils als Triplett), die sich über einen Konzentrationsbereich von 6 bis 7 Zehnerpotenzen erstrecken. Nach Zugabe von 50 mM TRIS-Puffer 50 mM pH 7,4, dem Radioliganden [³⁵S]GTPγS und der Membranpräparation, die mit Adenosindesaminase versetzt wurde, wird ein Endvolumen von 200 µl erreicht. Die Inkubation erfolgt 45 Minuten lang bei 25 °C im Schüttelwasserbad. Daraufhin wird die Reaktion zunächst mit 2 mL kaltem Waschpuffer (50 mM TRIS, 5 mM MgCl₂ × 2 H₂O, pH 7,4) abgestoppt und die Suspension über GF/B Filter mit Hilfe des Harvesters filtriert. Die Reagenzgläser werden zweimal mit je 2 ml kaltem Waschpuffer nachgespült. Die Flterscheiben werden mittels einer Ausstanzplatte in die Scintillationsvials überführt, die mit 2 ml Scintillationscocktail befüllt werden. Die Vials werden gut geschüttelt, um die Filterpapierchen vollständig zu benetzen. Nach einer Inkubation von mindestens 3 Stunden wird die Radioaktivität im LS-Counter durch eine 2minütige Messung bestimmt. Die Kurven werden jeweils dreimal reproduziert.

Im Assay werden die Lösungen der Testsubstanzen 1:20 verdünnt; der DMSO-Gehalt im Assay beträgt 5% (V/V).

### Auswertung der Radioligandbindungsstudien:

Aus drei unabhängigen Experimenten wird der Mittelwert berechnet. Aus der Cheng-Prusoff-Gleichung, der Konzentration und dem K_{D}-Wert des Radioliganden werden IC₅₀- und Kᵢ-Wert erhalten. Zur Berechnung wurde das Programm GraphPadPrism™, Version 3.0 (GraphPad, San Diego, California, USA) verwendet.

Resultate der biologischen Untersuchung ausgewählter Verbindungen:

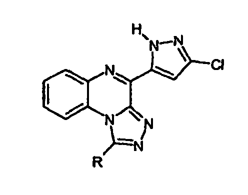

**Tabelle 1 : A₁ and A_{2A} Adenasin Rezeptoraffinitäten [aus : B. Matuszczak, E. Pekala, C.E. Müller, Arch. Pharm. Med. Chem. 331, 163-169 (1998)]**

| **Verbindung** | **A**_{**1**} ***K***_{**i**} **[µm] bzw. *(% Inhib. @ 10 µm)*** | **A**_{**2A**} **K**_{**i**} **[µM] bzw. *(% Inhib. @ 25 µM)*** |
|---|---|---|
| Coffein | 23,5 ± 3,0 | 32,5 |
| R = -H | >10 | >25 |
| | *(35%)* | *(21%)* |
| R = -CH3 | 7,85 ± 0,95 | 1,43 |
| R = -C6H5 | 1,62 ± 0,68 | >25 |
| | | *(0%)* |
| R = -CH2-C6H5 | 0,31 ± 0,09 | >25 |
| | | *(6,1% ± 1,5%)* |
| R = 2-furyl | 0,41 ± 0,16 | >25 |
| | | *(31% ± 3%)* |
| R = 2-thienyl | 0,71 ± 0,06 | >25 |
| | | *(22% ± 13%)* |
| R = 2-thienylmethyl | 0,20 ± 0,01 | >25 |
| | | *(21% ± 10%)* |

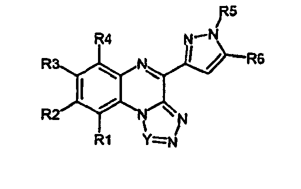

**Tabelle 2 : Rezeptoraffinitäten einiger neuer Triazolo- und Tetrazolochinoxalin-Derivate (t = nicht getestet)**

| **Y** | **für R1/2/3/4 ≠ H** | **R5** | **R6** | **A**_{**1**}***K***_{**i**} ***[µm] bzw. (% Inhib.@ 10µm)*** | **A**_{**2A**} ***K***_{**i**} **[µm] bzw. *(% Inhib.@ 10µm)*** | **A**_{**2B**} ***K***_{**i**} **[µm] bzw. *(% Inhib.@ 1µm)*** | **A**_{**3**} ***K***_{**i**} **[µm] bzw. (*% Inhib.@ 1µm)*** |
|---|---|---|---|---|---|---|---|
| C-CH₂-(2-thienyl) | R3 = OCH₃ | H | Cl | 0,90 ± 0,23 | 4,96 ± 1,29 | >1 | 0,0019 |
| | | | | | | *(0%)* | |
| C-CH₂-CH₂-CH₂-Ph | | H | Cl | 0,006 ± 0,001 | >10 | >1 | >1 |
| | | | | | *(6%)* | *(18%)* | *(32%)* |
| | | | | human : | | | |
| | | | | 2nM | | | |
| C-CH₂-O-CH₂-Ph | | H | Cl | 0,62 ± 0,22 | >10 | nt | nt |
| | | | | | (3%) | | |
| C-CH-(CH₃)-O-Ph | | H | Cl | 0,10 ± 0,03 | 1,18 ± 0,59 | nt | nt |
| C-(3-furyl) | | H | Cl | 0,32 ± 0,07 | 1,26 ± 0,18 | >1 | >1 |
| | | | | | | *(25%)* | *(31%)* |
| C-(3-thienyl) | | H | Cl | 0,33 ± 0,08 | 4,10 ± 1,50 | nt | nt |
| C-H | | H | Cl | >10 | >10 | >1 | 0,142 |
| | | | | *(35%)* | *(21%)* | *(0%)* | |
| C-H | R3 = OCH₃ | H | Cl | >10 | >10 | >1 | 0,372 |
| | | | | *(13%)* | *(1%)* | *(8%)* | |
| C-CH₂-(2-thienyl) | | CH₃ | Cl | 0,21 ± 0,04 | 8,61 ± 3,25 | >1 | 0,086 |
| | | | | | | *(22%)* | |
| N | | H | Cl | 0,67 ± 0,06 | 6,13 ± 1,38 | >1 | >1 |
| | | | | | | *(13%)* | *(53%)* |

### Dosierungen und Verabreichungsformen

Die erfindungsgemäßen Verbindungen der allgemeinen Formeln (1) oder (2) können Patienten peroral, beispielsweise mittels Tabletten, Dragees, Kapseln und Trinklösungen, rektal, beispielweise mittels Suppositorien, inhalativ, beispielsweise durch Einatmen von Aerosolen mit definierter Konzentration und Größenverteilung der Partikel, transdermal, beispielsweise durch wirkstoffhaltige Pflaster, Einreibelösungen, Gele usw., transmucosal, beispielsweise im Sinne einer Resorption durch die Mund- und Nasenschleimhaut, wobei der Wirkstoff in der Mundhöhle durch Lösung im Speichel freigesetzt wird, oder durch Sprühlösungen und dergleichen in die Nase eingebracht wird, mittels implantierter Behältnisse, die beispielsweise den Wirkstoff passivosmotisch oder gesteuert mittels Minipumpen oder dgl. freisetzen, durch intravenöse, intramuskuläre oder subkutane injektion und intrazerebroventrikulär verabreicht werden.

Typische Dosierungen bei Verabreichung dieser Wirkstoffe hängen von der Art der verwendeten Verbindung ab und liegen bei intravenöser Applikation für einen durchschnittlichen Erwachsenen im Bereich von 0,5 - 100 mg (vorzugsweise 1 - 50 mg), für perorale Applikation im Bereich von 1 - 1000 mg (vorzugsweise 5 - 500 mg), und für transdermale Applikation im Bereich von 0,5 - 50 mg (vorzugsweise 1 - 20 mg).
Bedingt durch das variable Verabreichungsspektrum eignen sich die erfindungsgemäßen Verbindungen der allgemeinen Formeln (1) oder (2) zur Herstellung von Arzneimitteln zur Behandlung von Erkrankungen im Bereich der Nieren, wie bei akutem Nierenversagen, Nephritis, hepatorenalem Syndrom, zur Behandlung des Herzens, vorzugsweise zur Behandlung von Herzrhythmusstörungen, Ischämie, Herzinfarkt bzw. Angina pectoris, zur Behandlung des zentralen Nervensystem (ZNS), vorzugsweise zur Behandlung von Demenz, Morbus Alzheimer, Angststörungen, Epilepsie, Morbus Parkinson, Schlaganfall, Depressionen, Opiatentzug oder komatösen Zuständen, oder zur Behandlung der Lunge, vorzugsweise zur Behandlung von Atemwegserkrankungen, wie Asthma, Bronchitis und Mucoviscidose, zur Behandlung von Hypertonie, allergischen Hauterkrankungen, wie Urticaria, oder Entzündungen.

Weiters können aus den erfindungsgemäßen Verbindungen (1) oder (2) Immunstimmulantia sowie Protektiva, welche bei Lungentransplantationen zum Einsatz kommen, hergestellt werden.

## Patentansprüche

1. Pyrazolyl-substituierte [1,2,4]Triazolo[4,3-*a*]chinoxaline gemäß Formel (**1**) 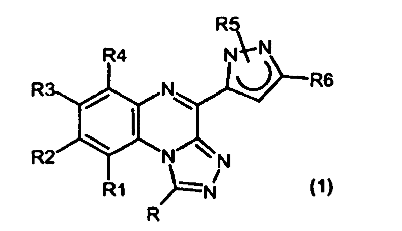
worin R1 bis R4 Wasserstoff, lineare oder verzweigte, gesättigte oder ungesättigte Alkylreste, Cycloalkylreste, welche gegebenenfalls ein oder mehrere Heteroatome aufweisen, Alkoxy-, Hydroxy-, Halogen-, Amino-, Nitro-, Trihalogenmethyl-, Carboxy-, Alkoxycarbonyl- oder Sulfogruppen sind, wobei R1 bis R4 identisch oder verschieden vorliegen können und worin der Substituent R Wasserstoff oder ein linearer oder verzweigtkettiger, gesättigter, und/oder ungesättigter Kohlenstoffrest, ein Cycloalkyl-Rest, ein Phenyl-, Pyridyl-, Thienyl- oder Furylrest in unsubstituierter oder ein- bzw. mehrfach substituierter Form, wobei der Substituent R entweder direkt oder über eine Alkylengruppe, in welcher ein oder mehrere Kohlenstoffatome durch Heteroatome, wie Sauerstoff, Schwefel oder Stickstoff ersetzt sein können, am Grundkörper gebunden ist, und worin der Substituent R5 Wasserstoff, C₁ - C₈ Alkyl, Allyl oder Acyl ist und worin der Rest R6 ein Halogen oder Wasserstoff ist, ausgenommen Verbindungen mit R1 bis R5 gleich Wasserstoff, R6 gleich Chlor und R gleich Methyl, Phenyl, Benzyl, 2-Furyl, 2-Thienyl oder 2-Thienylmethyl.

2. Verfahren zur Herstellung von Pyrazolyl-[1,2,4]triazolo[4,3-*a*]chinoxalinen gemäß Formel **(1), dadurch gekennzeichnet, dass** man substituierte 1,2 Diazine gemäß Formel **(C)** 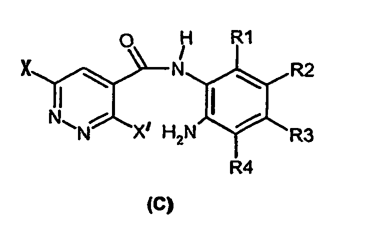
in welchen die Reste R1, R2, R3. R4 die Bedeutung gemäß Anspruch 1 haben und worin X' eine Abgangsgruppe und X Halogen oder Wasserstoff sind, durch Ringschluss zu pyrazolylsubstituierten Chinoxalinen gemäß Formel (**D**) 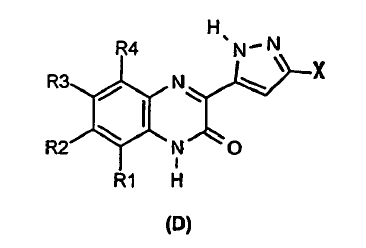
umsetzt, dass man anschließend die 2-Chlorchinoxalin-Derivate gemäß Formel (**E**) 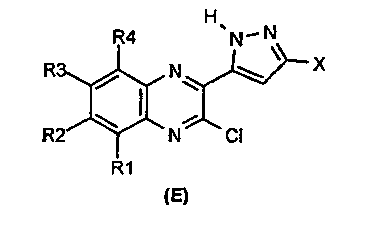
herstellt, welche man in die entsprechenden 2-Hydrazinoderivate gemäß Formel (**F**) 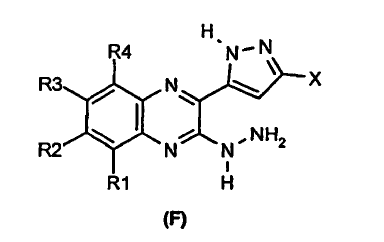
umsetzt, aus welchen durch Acylierung die Verbindungen gemäß Formel (**G**) 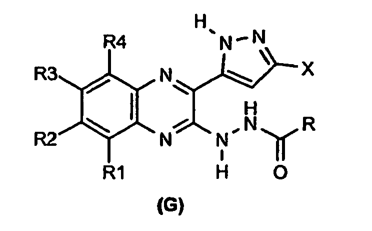
erhalten werden, aus welchen man durch Ringschlussreaktion die Pyrazolyl[1,2,4]triazolo-[4,3-*a*]chinoxaline gemäß Formel (**1**) erhält.

3. Verfahren nach Anspruch 2, **dadurch gekennzeichnet, dass** die Ringschlußreaktion ausgehend von dem entsprechenden Hydrazin ohne Isolierung des Hydrazid-Zwischenproduktes erfolgt.

4. Verfahren nach Anspruch 2 oder 3, **dadurch gekennzeichnet, dass** man die Verbindungen gemäß Formel (**G**) aus substituierten 2-Chlorchinoxalinen durch Umsetzung mit Hydraziden erhält.

5. Verfahren zur Herstellung symmetrisch substituierter Pyrazolyl[1,2,4]triazolo[4,3-*a*]chinoxaline nach einem der Ansprüche 2 bis 4, **dadurch gekennzeichnet, dass** man substituiertes Diazin gemäß Formel (**C**) nach Anspruch 2 durch Umsetzen von o-Phenylendiamin gemäß Formel (**B**), 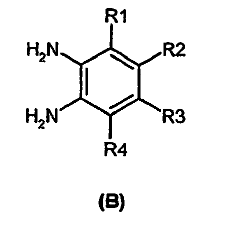
in welcher die Reste R1 bis R4 die Bedeutung gemäß Anspruch 1 haben, mit aktivierter 3,6-Dihalogenpyridazin-4-carbonsäure erhält.

6. Verfahren zur Herstellung von unsymmetrisch substituierten Pyrazolyl[1,2,4]triazolo[4,3-*a*]-chinoxalinen gemäß Formel (**1**), **dadurch gekennzeichnet, dass** man substituierte Diazine gemäß Formel (**C**), wie in Anspruch 2 dargestellt, durch *N*-Acylierung der o-Nitroanilin-Derivate gemäß Formel (**H**) 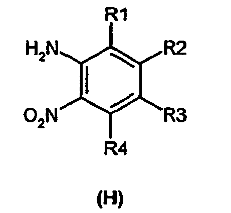
mit 3,6-Dihalogenpyridazin-4-carbonsäurechlorid unter Bildung der Verbindung gemäß Formel (**J**) 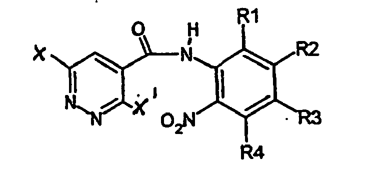
erhält, in welcher durch Reduktion der Nitrogruppe die Amingruppe in der Verbindung gemäß Formel **(C)** erhalten wird.

7. Verfahren zur Herstellung unsymmetrisch substituierter tricyclischer Verbindungen gemäß Formel (**1**), **dadurch gekennzeichnet sind, dass** ein unsymmetrisch substituiertes Phenylendiamin mit einem oder mehreren die Reaktivität beeinflussenden Substitutenten eingesetzt wird, so dass zumindest eine der beiden Aminogruppierungen für eine Acylierungsreaktion aktiviert oder deaktiviert wird, wodurch eine selektive Substitution am Ringsystem/an den Ringsystemen erfolgt.

8. Pyrazolyl-substituierte Tetrazolo[1,5-*a*]chinoxaline gemäß Formel (**2**) 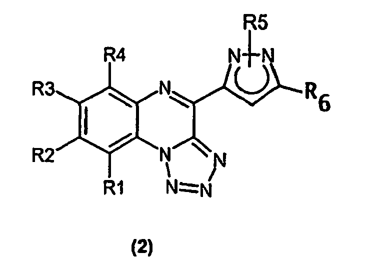
worin R 1 bis R6 sowie R die in Anspruch 1 angegebene Bedeutung haben.

9. Verfahren zur Darstellung von Verbindungen gemäß der allgemeinen Formeln (**1**) oder (**2**) 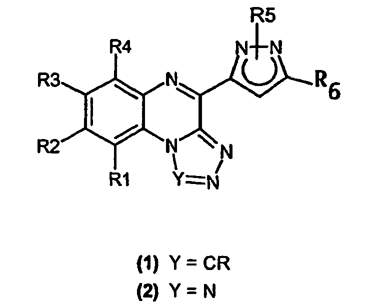
mit R5 ungleich Wasserstoff sowie die Trennung der anfallenden Isomeren, **dadurch gekennzeichnet, dass** entweder eine Verbindung der Formel (**1**) oder (**2**) mit R5 gleich Wasserstoff alkyliert wird oder dass die Enführung des Substituenten R5 bereits auf der Stufe der Verbindungen (**D**), (**E**), (**F**) oder (**G**), wie in Anspruch 2 angeführt, erfolgt, wobei die Umsetzung des 2-Chlorchinoxalins (**E**) bevorzugt ist.

10. Verfahren zur Herstellung der Verbindung gemäß Formel (**2**) aus Patentanspruch 9, **dadurch gekennzeichnet, dass** man die Verbindung der Formel (**E**), wie in Anspruch 2 angegeben, herstellt und mit Salzen der Stickstoffwasserstoffsäure umsetzt.

11. Pyrazolyl[1,2,4]triazolo[4,3-*a*]chinoxaline gemäß Formel (**1**) oder Pyrazolyl-substituierte Tetrazolo[1,5-*a*]chinoxaline gemäß Formel (**2**) isoliert oder in Form ihrer pharmazeutisch verträglichen Salze und Solvate als pharmazeutischer Wirkstoff, insbesondere als Adenosin-Rezeptor-Ligand.

12. Arzneimittel, **dadurch gekennzeichnet, dass** es als Wirkstoff Pyrazolyl-substituierte [1,2,4]Triazolo[4,3-*a*]chinoxaline gemäß Formel (1) und/oder Pyrazolyl-substituierte Tetrazolo[1,5-*a*]chinoxaline gemäß Formel (**2**) und/oder deren pharmazeutisch verträgliche Salze enthält.

13. Arzneimittel nach Anspruch 12, **dadurch gekennzeichnet, dass** es in einer für die perorale, rektale, inhalative, transdermale, transmucosale, intracerebroventrikuläre Verabreichung geeigneten Form vorliegt.

14. Arzneimittel nach Anspruch 12, **dadurch gekennzeichnet, dass** es in einer für Implantate, Infusionen oder Injektionen geeigneten Verabreichungsform vorliegt.

15. Verfahren zur Herstellung eines Arzneimittels nach einem der Ansprüche 12 bis 14 zur Behandlung von Erkrankungen im Bereich der Nieren, wie bei akutem Nierenversagen, Nephritis, hepatorenalem Syndrom, zur Behandlung des Herzens, vorzugsweise zur Behandlung von Herzrhythmusstörungen, lschämie, Herzinfarkt bzw. Angina pectoris, zur Behandlung des zentralen Nervensystem (ZNS), vorzugsweise zur Behandlung von Demenz, Morbus Alzheimer, Angststörungen, Epilepsie, Morbus Parkinson, Schlaganfall, Depressionen, Opiatentzug oder komatösen Zuständen, oder zur Behandlung der Lunge, vorzugsweise zur Behandlung von Atemwegserkrankungen, wie Asthma, Bronchitis und Mucoviscidose.

16. Verfahren zur Herstellung eines Arzneimittels nach einem der Ansprüche 12 bis 14, **dadurch gekennzeichnet, dass** als Arzneimittel Protetektiva, welche bei Lungentransplantationen verwendbar sind, hergestellt werden.

17. Verfahren zur Herstellung eines Arzneimittels nach einem der Ansprüche 12 bis 14 zur Behandlung von Hypertonie, allergischen Hauterkrankungen, wie Urticaria, oder Entzündungen.

18. Verfahren zur Herstellung eines Arzneimittels nach einem der Ansprüche 12 bis 14 , **dadurch gekennzeichnet, dass** als Arzneimittel ein lmmunstimulans hergestellt wird.

## Claims

1. Pyrazolyl-substituted [1,2,4]triazolo[4,3-*a*]quinoxalines according to formula (**1**) 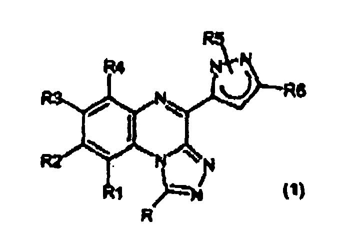
in which R1 to R4 are hydrogen, linear or branched, saturated or unsaturated alkyl radicals, cycloalkyl radicals, which if necessary have one or more heteroatoms, alkoxy, hydroxy, halogen, amino, nitro, trihalogenomethyl, carboxy, alkoxycarbonyl or sulphonic groups, R1 to R4 being able to be present identically or differently and in which the substituent R is hydrogen or a linear or branched-chain, saturated and/or unsaturated carbon radical, a cycloalkyl radical, a phenyl, pyridyl, thienyl or furyl radical in unsubstituted or once or repeatedly substituted form, the substituent R being connected to the basic body either directly or via an alkylene group, in which one or more carbon atoms can be replaced by heteroatoms such as oxygen, sulphur or nitrogen, and in which the substituent R5 is hydrogen, C₁-C₈ alkyl, allyl or acyl and in which the radical R6 is a halogen or hydrogen, with the exception of compounds with R1 to R5 the same as hydrogen, R6 the same as chlorine and R the same as methyl, phenyl, benzyl, 2-furyl, 2-thienyl or 2-thienylmethyl.

2. Method for producing pyrazolyl-[1,2,4]triazolo[4,3-*a*]quinoxalines according to formula (1), **characterised in that** substituted 1,2diazines according to formula (**C**) 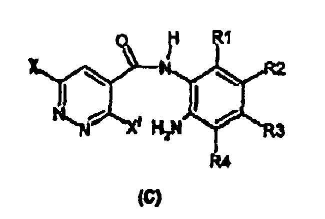
in which the radicals R1, R2, R3, R4 have the meaning according to claim 1, and in which X' is a nucleofuge and X is halogen or hydrogen, are converted by means of ring closure into pyrazolyl-substituted quinoxalines according to formula (**D**) 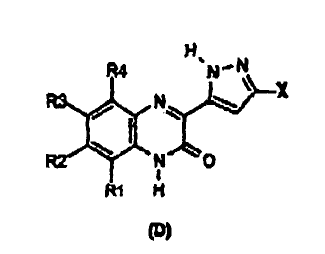
and **in that** the 2-chloroquinoxaline derivatives according to formula (**E**) are next produced 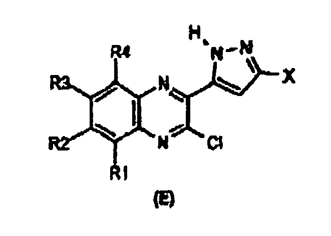
which are converted into the corresponding 2-hydrazino derivatives according to formula (**F**) 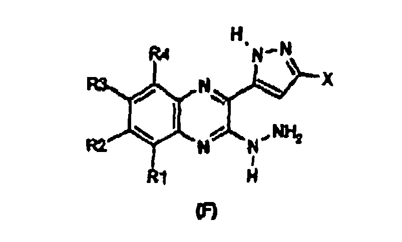
from which, by means of acylation, the compounds according to formula (**G**) 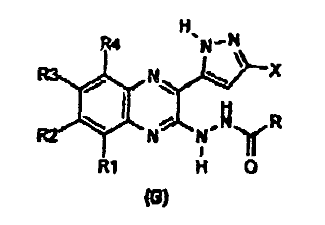
are obtained, from which, by means of ring closure reaction, the pyrazolyl[1,2,4]triazolo[4,3-*a*]quinoxalines according to formula (**1**) are obtained.

3. Method according to claim 2, **characterised in that** the ring closure reaction is effected starting from the corresponding hydrazine without isolation of the hydrazide intermediate product.

4. Method according to claim 2 or 3, **characterised in that** the compounds according to formula (**G**) are obtained from substituted 2-chloroquinoxalines by means of conversion with hydrazides.

5. Method for producing symmetrically substituted pyrazolyl[1,2,4]triazolo[4,3-*a*]quinoxalines according to one of the claims 2 to 4, **characterised in that** substituted diazine according to formula (C) according to claim 2 is obtained by means of conversion of o-phenylenediamine according to formula (**B**) 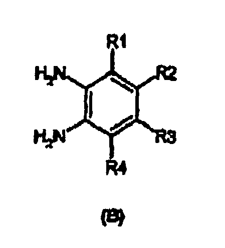
in which the radicals R1 to R4 have the meaning according to claim 1, with activated 3,6-dihalogenpyridazine-4-carboxylic acid.

6. Method for producing asymmetrically substituted pyrazolyl[1,2,4]triazolo[4,3-*a*]quinoxalines according to formula (**1**), **characterised in that** substituted diazines according to formula (**C**), as shown in claim 2, are obtained by means of *N*-acylation of the o-nitroaniline derivatives according to formula **(H)** 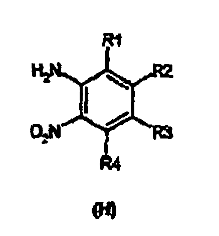
with 3,6-dihalogenopyridazine-4-carboxylic acid chloride with formation of the compound according to formula (**J**) 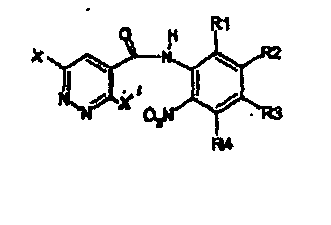
in which the amine group in the compound according to formula (C) is obtained by means of reduction of the nitro group.

7. Method for producing asymmetrically substituted tricyclic compounds according to formula (**1**), **characterised in that** an asymmetrically substituted phenylenediaznine with one or more substituents which influence the reactivity is used, so that at least one of the two amino groupings is activated or deactivated for an acylation reaction, as a result of which a selective substitution on the ring system/systems is effected.

8. Pyrazolyl-substituted tetrazolo[1,5-*a*]quinoxalines according to formula (**2**) 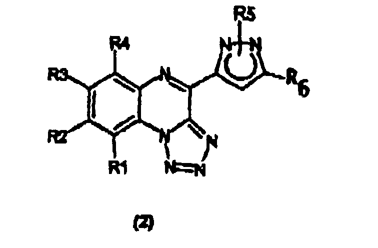
in which R1 to R6 and also R have the meaning indicated in claim 1.

9. Method for preparing compounds according to the general formulae (**1**) or (**2**) 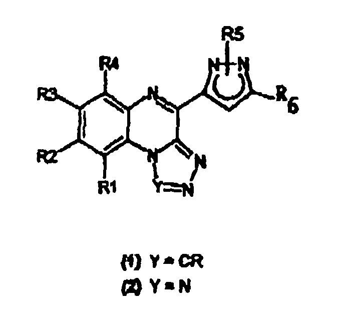
with R5 not the same as hydrogen and the separation of the obtained isomers, **characterised in that** either a compound of formula (**1**) or (**2**) with R5 the same as hydrogen is acylated or **in that** the introduction of the substituent R5 is already effected at the stage of the compounds (**D**), (**E**), (**F**) or (**G**), as cited in claim 2, the conversion of 2-chlorquinoxaline (E) being preferred.

10. Method for producing the compound according to formula (**2**) from patent claim 9, **characterised in that** the compound of formula (**E**), as indicated in claim 2, is produced and converted with salts of hydrazoic acid.

11. Pyrazolyl[1,2,4]triazolo[4,3-*a*]quinoxalines according to formula (**1**) or pyrazolyl-substituted tetrazolo[1,5-*a*]quinoxalines according to formula (**2**), isolated or in the form of their pharmaceutically compatible salts and solvates as pharmaceutical active substance, in particular as adenosine receptor ligand.

12. Drug, **characterised in that** it contains pyrazolyl-substituted [1,2,4]triazolo[4,3-*a*]quinoxalines according to formula (**1**) and/or pyrazolyl-substituted tetrazolo[1,5-*a*]quinoxalines according to formula (**2**) and/or the pharmaceutically compatible salts thereof as active substance.

13. Drug according to claim 12, **characterised in that** it is present in a form suitable for peroral, rectal, inhalative, transdermal, transmucosal, intracerebroventricular administration.

14. Drug according to claim 12, **characterised in that** it is present in an administration form suitable for implants, infusions or injections.

15. Method for producing a drug according to one of the claims 12 to 14, for the treatment of diseases in the field of kidneys, such as in acute kidney failure, nephritis, hepatorenal syndrome, for the treatment of the heart, preferably for the treatment of heart rhythm disorders, ischaemia, heart infarct or angina pectoris, for the treatment of the central nervous system (CNS), preferably for the treatment of dementia, Alzheimer's disease, anxiety disorders, epilepsy, Parkinson's disease, stroke, depressions, opiate withdrawal or comatose states, or for the treatment of the lung, preferably for the treatment of diseases of the airways, such as asthma, bronchitis and mucoviscidosis.

16. Method for producing a drug according to one of the claims 12 to 14, **characterised in that** protectiva, which can be used in lung transplantations, are produced as a drug.

17. Method for producing a drug according to one of the claims 12 to 14, for the treatment of hypertonia, allergic skin diseases, such as urticaria, or inflammations.

18. Method for producing a drug according to one of the claims 12 to 14, **characterised in that** an immune stimulant is produced as a drug.

## Revendications

1. [1,2,4]triazolo[4,3-a]quinoxalines portant un ou plusieurs substituants pyrazolyle, répondant à la formule (1) 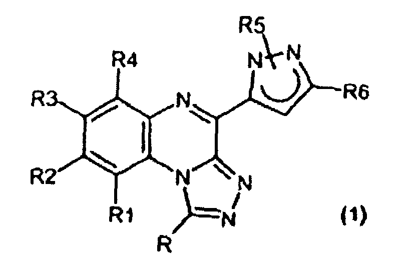
dans laquelle R1 à R4 représentent un atome d'hydrogène, des radicaux alkyle saturés ou insaturés, linéaires ou ramifiés, des radicaux cycloalkyle qui peuvent éventuellement présenter un ou plusieurs hétéroatomes, un groupe alcoxy, un groupe hydroxyle, un atome d'halogène, un groupe amino, un groupe nitro, un groupe trihalogénométhyle, un groupe carboxyle, un groupe alcoxycarbonyle ou un groupe sulfo, R1 à R4 pouvant être présents sous forme identique ou sous forme différente, et dans laquelle le substituant R représente un atome d'hydrogène ou un radical d'hydrocarbure saturé et/ou insaturé, à chaîne linéaire ou ramifiée, un radical cycloalkyle, un radical phényle, un radical pyridyle, un radical thiényle ou un radical furyle sous forme non substituée ou sous forme une fois, respectivement plusieurs fois substituée, le substituant R étant lié au corps de base de manière directe ou via un groupe alkylène dans lequel un ou plusieurs atomes de carbone peuvent être remplacés par des hétéroatomes tels qu'un atome d'oxygène, un atome de soufre ou un atome d'azote, et dans laquelle le substituant R5 représente un atome d'hydrogène, un groupe alkyle en C₁-C₈, un groupe allyle ou un groupe acyle, et dans laquelle le radical R6 représente un atome d'halogène ou un atome d'hydrogène, à l'exception des composés dans lesquels R1 à R5 représentent un atome d'hydrogène, R6 représente un atome de chlore et R représente un groupe méthyle, un groupe phényle, un groupe benzyle, un groupe 2-furyle, un groupe 2-thiényle ou un groupe 2-thiényl-méthyle.

2. Procédé pour la préparation de pyrazolyl-[1,2,4]triazolo[4,3-a]-quinoxalines répondant à la formule (**1**), **caractérisé en ce qu'**on fait réagir des 1,2-diazines substituées répondant à la formule (**C**) 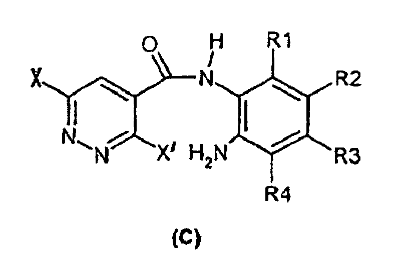
dans lesquelles les radicaux R1, R2, R3, R4 ont la signification donnée à la revendication 1, et dans lesquelles X' représente un groupe sortant et X représente un atome d'halogène ou un atome d'hydrogène, par cyclisation pour obtenir des quinoxalines à substitution pyrazolyle répondant à la formule (**D**) 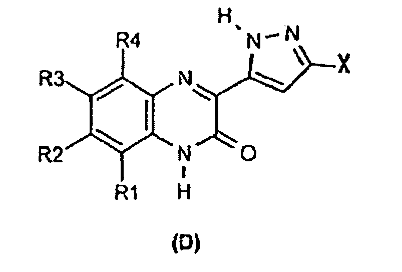 en ce qu'on prépare ensuite les dérivés 2-chloroquinoxaline répondant à la formule (**E**) 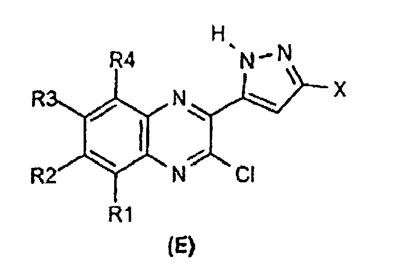 que l'on fait réagir pour obtenir les dérivés 2-hydrazino correspondants répondant à la formule (**F**) 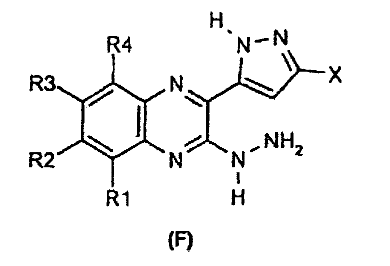 à partir desquels on obtient, par acylation, les composés répondant à la formule (**G**) 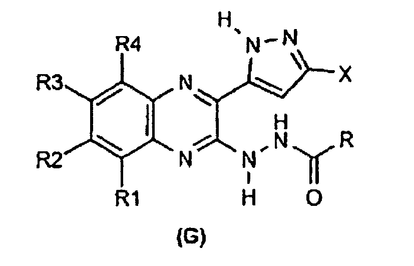 à partir desquels on obtient, via une réaction de cyclisation, les pyrazolyl-[1,2,4]triazolo[4,3-a]quinoxalines répondant à la formule (1).

3. Procédé selon la revendication 2, **caractérisé en ce que** la réaction de cyclisation a lieu à partir de l'hydrazine correspondante sans isolation du produit intermédiaire d'hydrazide.

4. Procédé selon la revendication 2 ou 3, **caractérisé en ce qu'**on obtient les composés répondant à la formule (**G**) à partir de 2-chloroquinoxalines substituées, par mise en réaction avec des hydrazides.

5. Procédé pour la préparation de pyrazolyl[1,2,4]triazolo[4,3-a]-quinoxalines soumises à une substitution symétrique, selon l'une quelconque des revendications 2 à 4, **caractérisé en ce qu'**on obtient de la diazine substituée répondant à la formule (**C**) selon la revendication 2 par mise en réaction de o-phénylènediamine répondant à la formule (**B**) 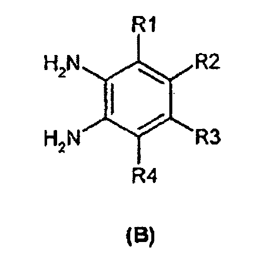
dans laquelle les radicaux R1 à R4 ont la signification indiquée à la revendication 1, avec de l'acide 3,6-dihalogénopyridazine-4-carboxylique activé.

6. Procédé pour la préparation de pyrazolyl[1,2,4]triazolo[4,3-*a*]-quinoxalines soumises à une substitution asymétrique répondant à la formule (**1**), **caractérisé en ce qu'**on prépare des diazines substituées répondant à la formule (**C**), comme indiqué à la revendication 2, par *N-*acylation des dérivés de o-nitroaniline répondant à la formule (**H**) 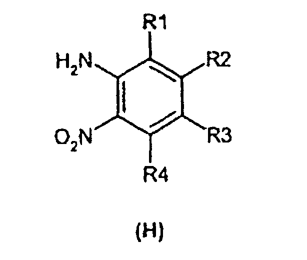 avec du chlorure d'acide 3,6-dihalogénopyridazine-4-carboxylique, avec formation du composé répondant à la formule (**J**), 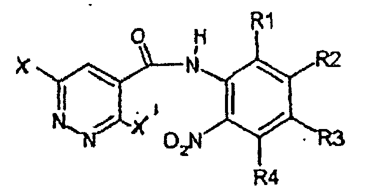 le groupe amine dans le composé répondant à la formule (**C**) étant obtenu par réduction du groupe nitro.

7. Procédé pour la préparation de composés tricycliques soumis à une substitution asymétrique répondant à la formule (**1**), **caractérisé en ce qu'**on met en oeuvre une phénylènediamine soumise à une substitution asymétrique avec un ou plusieurs substituants influençant la réactivité, si bien que l'on obtient une activation ou une désactivation d'au moins un des deux groupements amino pour une réaction d'acylation, donnant lieu à une substitution sélective sur le système cyclique/sur les systèmes cycliques.

8. Tétrazolo[1,5-a]quinoxalines portant un ou plusieurs substituants pyrazolyle, répondant à la formule (**2**) 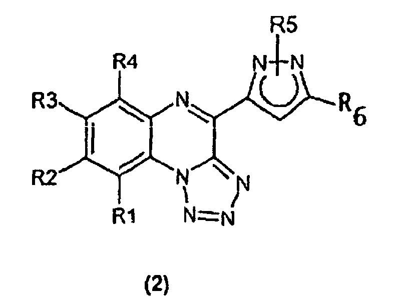
dans laquelle R1 à R6 et R ont la signification indiquée à la revendication 1.

9. Procédé pour la préparation de composés répondant à la formule générale (**1**) ou (**2**) 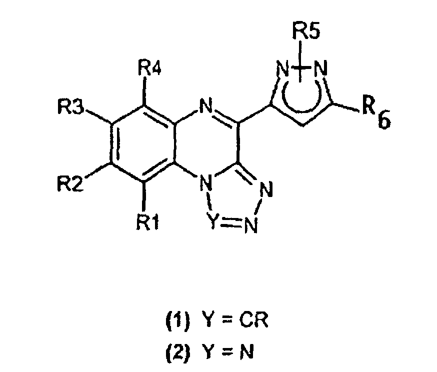
dans lesquelles R5 ne représente pas un atome d'hydrogène et pour la séparation des isomères obtenus, **caractérisé en ce que**, soit on soumet à une alkylation un composé répondant à la formule (**1**) ou (**2**) dans laquelle R5 représente un atome d'hydrogène, soit on introduit le substituant R5, déjà au stade de préparation des composés (**D**), (**E**), (**F**) ou (**G**), comme indiqué à la revendication 2, la mise en réaction de la 2-chloroquinoxaline (**E**) étant préférée.

10. Procédé pour la préparation du composé répondant à la formule (2) selon la revendication 9, **caractérisé en ce qu'**on prépare le composé répondant à la formule (**E**), comme indiqué à la revendication 2, et on le fait réagir avec des sels de l'acide azothydrique.

11. Pyrazolyl[1,2,4]triazolo[4,3-*a*]-quinoxalines répondant à la formule (**1**) ou tétrazolo[1,5-*a*]quinoxalines portant un ou plusieurs substituants pyrazolyle, répondant à la formule (**2**) sous forme isolée ou sous forme de leurs sels et de leurs solvates pharmaceutiquement acceptables, à titre de substance active pharmaceutique, en particulier à titre de ligand du récepteur de l'adénosine.

12. Médicament, **caractérisé en ce qu'**il contient, à titre de substance active, des [1,2,4]triazolo[4,3-a]-quinoxalines portant un ou plusieurs substituants pyrazolyle répondant à la formule (**1**) et/ou des tétrazolo[1,5-a]quinoxalines portant un ou plusieurs substituants pyrazolyle répondant à la formule (**2**) et/ou leurs sels pharmaceutiquement acceptables.

13. Médicament selon la revendication 12, **caractérisé en ce qu'**il est présent sous une forme appropriée pour l'administration par voie orale, par voie rectale, par inhalation, par voie transdermique, par voie transmuqueuse, par voie intracérébroventriculaire.

14. Médicament selon la revendication 12, **caractérisé en ce qu'**il est présent sous une forme d'administration appropriée pour des implants, pour des perfusions ou pour des injections.

15. Procédé pour la préparation d'un médicament selon l'une quelconque des revendications 12 à 14 pour le traitement de maladies dans le domaine des reins, comme par exemple la défaillance rénale aiguë, la néphrite, le syndrome hépatorénal, pour le traitement du coeur, de préférence pour le traitement de troubles du rythme cardiaque, de l'ischémie, de l'infarctus du myocarde, respectivement de l'angine de poitrine, pour le traitement du système nerveux central (SNC), de préférence pour le traitement de la démence, de la maladie d'Alzheimer, des troubles de l'anxiété, de l'épilepsie, de la maladie de Parkinson, de l'apoplexie cérébrale, des dépressions, du sevrage des opiacés, ou encore d'états comateux, ou pour le traitement des poumons, de préférence pour le traitement de maladies des voies respiratoires, telles que l'asthme, la bronchite et la mucoviscidose.

16. Procédé pour la préparation d'un médicament selon l'une quelconque des revendications 12 à 14, **caractérisé en ce qu'**on prépare, à titre de médicament, des agents protecteurs que l'on peut utiliser dans des transplantations pulmonaires.

17. Procédé pour la préparation d'un médicament selon l'une quelconque des revendications 12 à 14, pour le traitement de l'hypertonie, de maladies allergiques de la peau, telles que l'urticaire ou encore d'inflammations.

18. Procédé pour la préparation d'un médicament selon l'une quelconque des revendications 12 à 14, **caractérisé en ce qu'**on prépare, à titre de médicament, un immunostimulant.
